# EUROPEAN PATENT APPLICATION

(11) **EP 3 561 053 A1**
(43) Date of publication of application: **30.10.2019**
(21) Application number: 18169600.6
(22) Date of filing: 26.04.2018
(51) Int. Cl.: C12N 5/0783, C12N 5/10, C07K 19/00, A61K 35/17, A61K 39/00, A61P 35/00

(54) **IMMUNE EFFECTOR CELLS AND MOLECULAR ADAPTORS WITH AN ANTIGEN CYTOKINE COMPLEX FOR EFFECTIVE CANCER IMMUNOTHERAPY**

(71) Applicant: Baylor College of Medicine, Houston, TX 77030 (US)
(72) Inventor: METELITSA, Leonid S., Houston, TX 77030 (US); LIU, Bin, Houston, TX 77030 (US); JIN, Jingling, Houston, TX 77030 (US); LIU, Daofeng, Houston, TX 77025 (US)
(74) Representative: J A Kemp LLP

(57) **Abstract**

The disclosure concerns cancer immunotherapy and configuring the immunotherapy with modular molecular adaptors. In certain embodiments, there is an immune effector cell, wherein the cell produces a molecular adaptor comprising: (a) a tumor cell binding domain, and (b) a cytokine domain comprising (i) a cytokine or a functional fragment thereof; or (ii) a cytokine receptor or a functional fragment thereof; or (iii) a combination of both (i) and (ii); (c) optionally further comprising an antigen receptor target domain; optionally wherein the cell comprises one or more engineered antigen receptors against one or more tumor target antigens.

SEQUENCE LISTING

<110>  BAYLOR COLLEGE OF MEDICINE

<120>  IMMUNE EFFECTOR CELLS AND MOLECULAR ADAPTORS WITH AN ANTIGEN
       CYTOKINE COMPLEX FOR EFFECTIVE IMMUNOTHERAPY

<130>  N413515EP

<160>  15

<170>  PatentIn version 3.5

<210>  1
<211>  491
<212>  PRT
<213>  Artificial Sequence

<220>
<223>  CAR19

<400>  1

Met Glu Phe Gly Leu Ser Trp Leu Phe Leu Val Ala Ile Leu Lys Gly
1               5                   10                  15

Val Gln Cys Ser Arg Asp Ile Gln Met Thr Gln Thr Thr Ser Ser Leu
            20                  25                  30

Ser Ala Ser Leu Gly Asp Arg Val Thr Ile Ser Cys Arg Ala Ser Gln
        35                  40                  45

Asp Ile Ser Lys Tyr Leu Asn Trp Tyr Gln Gln Lys Pro Asp Gly Thr
    50                  55                  60

Val Lys Leu Leu Ile Tyr His Thr Ser Arg Leu His Ser Gly Val Pro
65                  70                  75                  80

Ser Arg Phe Ser Gly Ser Gly Ser Gly Thr Asp Tyr Ser Leu Thr Ile
                85                  90                  95

Ser Asn Leu Glu Gln Glu Asp Ile Ala Thr Tyr Phe Cys Gln Gln Gly
            100                 105                 110

Asn Thr Leu Pro Tyr Thr Phe Gly Gly Gly Thr Lys Leu Glu Leu Lys
        115                 120                 125

Arg Gly Gly Gly Gly Ser Gly Gly Gly Gly Ser Gly Gly Gly Gly Ser
    130                 135                 140

Gly Gly Gly Gly Ser Glu Val Gln Leu Gln Gln Ser Gly Pro Gly Leu
145                 150                 155                 160

Val Ala Pro Ser Gln Ser Leu Ser Val Thr Cys Thr Val Ser Gly Val
                165                 170                 175

Ser Leu Pro Asp Tyr Gly Val Ser Trp Ile Arg Gln Pro Pro Arg Lys
            180                 185                 190

Gly Leu Glu Trp Leu Gly Val Ile Trp Gly Ser Glu Thr Thr Tyr Tyr
        195                 200                 205

Asn Ser Ala Leu Lys Ser Arg Leu Thr Ile Ile Lys Asp Asn Ser Lys
    210                 215                 220

Ser Gln Val Phe Leu Lys Met Asn Ser Leu Gln Thr Asp Asp Thr Ala
225                 230                 235                 240

Ile Tyr Tyr Cys Ala Lys His Tyr Tyr Tyr Gly Gly Ser Tyr Ala Met
                245                 250                 255

Asp Tyr Trp Gly Gln Gly Thr Thr Val Thr Val Ser Ser Thr Thr Thr
            260                 265                 270

Pro Ala Pro Arg Pro Pro Thr Pro Ala Pro Thr Ile Ala Ser Gln Pro
        275                 280                 285

Leu Ser Leu Arg Pro Glu Ala Cys Arg Pro Ala Ala Gly Gly Ala Val
    290                 295                 300

His Thr Arg Gly Leu Asp Phe Ala Cys Asp Ile Tyr Ile Trp Ala Pro
305                 310                 315                 320

Leu Ala Gly Thr Cys Gly Val Leu Leu Leu Ser Leu Val Ile Thr Leu
                325                 330                 335

Tyr Cys Arg Ser Lys Arg Ser Arg Leu Leu His Ser Asp Tyr Met Asn
            340                 345                 350

Met Thr Pro Arg Arg Pro Gly Pro Thr Arg Lys His Tyr Gln Pro Tyr
        355                 360                 365

Ala Pro Pro Arg Asp Phe Ala Ala Tyr Arg Ser Arg Val Lys Phe Ser
    370                 375                 380

Arg Ser Ala Asp Ala Pro Ala Tyr Gln Gln Gly Gln Asn Gln Leu Tyr
385                 390                 395                 400

Asn Glu Leu Asn Leu Gly Arg Arg Glu Glu Tyr Asp Val Leu Asp Lys
                405                 410                 415

Arg Arg Gly Arg Asp Pro Glu Met Gly Gly Lys Pro Arg Arg Lys Asn
        420                 425                 430

Pro Gln Glu Gly Leu Tyr Asn Glu Leu Gln Lys Asp Lys Met Ala Glu
    435                 440                 445

Ala Tyr Ser Glu Ile Gly Met Lys Gly Glu Arg Arg Arg Gly Lys Gly
    450                 455                 460

His Asp Gly Leu Tyr Gln Gly Leu Ser Thr Ala Thr Lys Asp Thr Tyr
465                 470                 475                 480

Asp Ala Leu His Met Gln Ala Leu Pro Pro Arg
                485                 490

<210>  2
<211>  492
<212>  PRT
<213>  Artificial Sequence

<220>
<223>  CARGD2

<400>  2

Met Glu Phe Gly Leu Ser Trp Leu Phe Leu Val Ala Ile Leu Lys Gly
1               5                   10                  15

Val Gln Cys Ser Arg Asp Ile Leu Leu Thr Gln Thr Pro Leu Ser Leu
            20                  25                  30

Pro Val Ser Leu Gly Asp Gln Ala Ser Ile Ser Cys Arg Ser Ser Gln
        35                  40                  45

Ser Leu Val His Arg Asn Gly Asn Thr Tyr Leu His Trp Tyr Leu Gln
    50                  55                  60

Lys Pro Gly Gln Ser Pro Lys Leu Leu Ile His Lys Val Ser Asn Arg
65                  70                  75                  80

Phe Ser Gly Val Pro Asp Arg Phe Ser Gly Ser Gly Ser Gly Thr Asp
                85                  90                  95

Phe Thr Leu Lys Ile Ser Arg Val Glu Ala Glu Asp Leu Gly Val Tyr
            100                 105                 110

Phe Cys Ser Gln Ser Thr His Val Pro Pro Leu Thr Phe Gly Ala Gly
        115                 120                 125

Thr Lys Leu Glu Leu Lys Arg Ala Asp Ala Ala Pro Thr Val Ser Ile
    130                 135                 140

Phe Pro Gly Ser Gly Gly Gly Gly Ser Gly Gly Glu Val Lys Leu Gln
145                 150                 155                 160

Gln Ser Gly Pro Ser Leu Val Glu Pro Gly Ala Ser Val Met Ile Ser
                165                 170                 175

Cys Lys Ala Ser Gly Ser Ser Phe Thr Gly Tyr Asn Met Asn Trp Val
            180                 185                 190

Arg Gln Asn Ile Gly Lys Ser Leu Glu Trp Ile Gly Ala Ile Asp Pro
        195                 200                 205

Tyr Tyr Gly Gly Thr Ser Tyr Asn Gln Lys Phe Lys Gly Arg Ala Thr
    210                 215                 220

Leu Thr Val Asp Lys Ser Ser Ser Thr Ala Tyr Met His Leu Lys Ser
225                 230                 235                 240

Leu Thr Ser Glu Asp Ser Ala Val Tyr Tyr Cys Val Ser Gly Met Glu
                245                 250                 255

Tyr Trp Gly Gln Gly Thr Ser Val Thr Val Ser Ser Thr Arg Thr Thr
            260                 265                 270

Thr Pro Ala Pro Arg Pro Pro Thr Pro Ala Pro Thr Ile Ala Ser Gln
        275                 280                 285

Pro Leu Ser Leu Arg Pro Glu Ala Cys Arg Pro Ala Ala Gly Gly Ala
    290                 295                 300

Val His Thr Arg Gly Leu Asp Phe Ala Cys Asp Ile Tyr Ile Trp Ala
305                 310                 315                 320

Pro Leu Ala Gly Thr Cys Gly Val Leu Leu Leu Ser Leu Val Ile Thr
                325                 330                 335

Leu Tyr Cys Arg Ser Lys Arg Ser Arg Leu Leu His Ser Asp Tyr Met
            340                 345                 350

Asn Met Thr Pro Arg Arg Pro Gly Pro Thr Arg Lys His Tyr Gln Pro
        355                 360                 365

Tyr Ala Pro Pro Arg Asp Phe Ala Ala Tyr Arg Ser Arg Val Lys Phe
    370                 375                 380

Ser Arg Ser Ala Asp Ala Pro Ala Tyr Gln Gln Gly Gln Asn Gln Leu
385                 390                 395                 400

Tyr Asn Glu Leu Asn Leu Gly Arg Arg Glu Glu Tyr Asp Val Leu Asp
                405                 410                 415

Lys Arg Arg Gly Arg Asp Pro Glu Met Gly Gly Lys Pro Arg Arg Lys
            420                 425                 430

Asn Pro Gln Glu Gly Leu Tyr Asn Glu Leu Gln Lys Asp Lys Met Ala
        435                 440                 445

Glu Ala Tyr Ser Glu Ile Gly Met Lys Gly Glu Arg Arg Arg Gly Lys
    450                 455                 460

Gly His Asp Gly Leu Tyr Gln Gly Leu Ser Thr Ala Thr Lys Asp Thr
465                 470                 475                 480

Tyr Asp Ala Leu His Met Gln Ala Leu Pro Pro Arg
                485                 490


<210>  3
<211>  12
<212>  PRT
<213>  Homo sapiens

<400>  3

Glu Ser Lys Tyr Gly Pro Pro Cys Pro Ser Cys Pro
1               5                   10


<210>  4
<211>  272
<212>  PRT
<213>  Homo sapiens

<400>  4

Pro Glu Glu Pro Leu Val Val Lys Val Glu Glu Gly Asp Asn Ala Val
1               5                   10                  15

Leu Gln Cys Leu Lys Gly Thr Ser Asp Gly Pro Thr Gln Gln Leu Thr
            20                  25                  30

Trp Ser Arg Glu Ser Pro Leu Lys Pro Phe Leu Lys Leu Ser Leu Gly
        35                  40                  45

Leu Pro Gly Leu Gly Ile His Met Arg Pro Leu Ala Ile Trp Leu Phe
    50                  55                  60

Ile Phe Asn Val Ser Gln Gln Met Gly Gly Phe Tyr Leu Cys Gln Pro
65                  70                  75                  80

Gly Pro Pro Ser Glu Lys Ala Trp Gln Pro Gly Trp Thr Val Asn Val
                85                  90                  95

Glu Gly Ser Gly Glu Leu Phe Arg Trp Asn Val Ser Asp Leu Gly Gly
            100                 105                 110

Leu Gly Cys Gly Leu Lys Asn Arg Ser Ser Glu Gly Pro Ser Ser Pro
        115                 120                 125

Ser Gly Lys Leu Met Ser Pro Lys Leu Tyr Val Trp Ala Lys Asp Arg
    130                 135                 140

Pro Glu Ile Trp Glu Gly Glu Pro Pro Cys Leu Pro Pro Arg Asp Ser
145                 150                 155                 160

Leu Asn Gln Ser Leu Ser Gln Asp Leu Thr Met Ala Pro Gly Ser Thr
                165                 170                 175

Leu Trp Leu Ser Cys Gly Val Pro Pro Asp Ser Val Ser Arg Gly Pro
            180                 185                 190

Leu Ser Trp Thr His Val His Pro Lys Gly Pro Lys Ser Leu Leu Ser
        195                 200                 205

Leu Glu Leu Lys Asp Asp Arg Pro Ala Arg Asp Met Trp Val Met Glu
    210                 215                 220

Thr Gly Leu Leu Leu Pro Arg Ala Thr Ala Gln Asp Ala Gly Lys Tyr
225                 230                 235                 240

Tyr Cys His Arg Gly Asn Leu Thr Met Ser Phe His Leu Glu Ile Thr
                245                 250                 255

Ala Arg Pro Val Leu Trp His Trp Leu Leu Arg Thr Gly Gly Trp Lys
            260                 265                 270

<210>  5
<211>  20
<212>  PRT
<213>  thosea asigna virus 2A

<400>  5

Arg Ala Glu Gly Arg Gly Ser Leu Leu Thr Cys Gly Asp Val Glu Glu
1               5                   10                  15

Asn Pro Gly Pro
            20

<210>  6
<211>  19
<212>  PRT
<213>  porcine teschovirus-1 2A

<400>  6

Ala Thr Asn Phe Ser Leu Leu Lys Gln Ala Gly Asp Val Glu Glu Asn
1               5                   10                  15

Pro Gly Pro

<210>  7
<211>  20
<212>  PRT
<213>  Artificial Sequence

<220>
<223>  Linker 1

<400>  7

Gly Gly Gly Gly Ser Gly Gly Gly Gly Ser Gly Gly Gly Ser Leu Gln
1               5                   10                  15

Gly Thr Thr Cys
            20

<210>  8
<211>  26
<212>  PRT
<213>  Artificial Sequence

<220>
<223>  Linker 2

<400>  8

Ser Gly Gly Gly Ser Gly Gly Gly Gly Ser Gly Gly Gly Gly Ser Gly
1               5                   10                  15

Gly Gly Gly Ser Gly Gly Gly Ser Leu Gln
            20                  25

<210>  9
<211>  162
<212>  PRT
<213>  Homo sapiens

<400>  9

Met Arg Ile Ser Lys Pro His Leu Arg Ser Ile Ser Ile Gln Cys Tyr

1               5                   10                  15

Leu Cys Leu Leu Leu Asn Ser His Phe Leu Thr Glu Ala Gly Ile His
            20                  25                  30

Val Phe Ile Leu Gly Cys Phe Ser Ala Gly Leu Pro Lys Thr Glu Ala
        35                  40                  45

Asn Trp Val Asn Val Ile Ser Asp Leu Lys Lys Ile Glu Asp Leu Ile
    50                  55                  60

Gln Ser Met His Ile Asp Ala Thr Leu Tyr Thr Glu Ser Asp Val His
65                  70                  75                  80

Pro Ser Cys Lys Val Thr Ala Met Lys Cys Phe Leu Leu Glu Leu Gln
                85                  90                  95

Val Ile Ser Leu Glu Ser Gly Asp Ala Ser Ile His Asp Thr Val Glu
            100                 105                 110

Asn Leu Ile Ile Leu Ala Asn Asn Ser Leu Ser Ser Asn Gly Asn Val
        115                 120                 125

Thr Glu Ser Gly Cys Lys Glu Cys Glu Glu Leu Glu Glu Lys Asn Ile
    130                 135                 140

Lys Glu Phe Leu Gln Ser Phe Val His Ile Val Gln Met Phe Ile Asn
145                 150                 155                 160

Thr Ser

<210>  10
<211>  205
<212>  PRT
<213>  Homo sapiens

<400>  10

Met Ala Pro Arg Arg Ala Arg Gly Cys Arg Thr Leu Gly Leu Pro Ala
1               5                   10                  15

Leu Leu Leu Leu Leu Leu Leu Arg Pro Pro Ala Thr Arg Gly Ile Thr
            20                  25                  30

Cys Pro Pro Pro Met Ser Val Glu His Ala Asp Ile Trp Val Lys Ser
        35                  40                  45

Tyr Ser Leu Tyr Ser Arg Glu Arg Tyr Ile Cys Asn Ser Gly Phe Lys

50                  55                  60

Arg Lys Ala Gly Thr Ser Ser Leu Thr Glu Cys Val Leu Asn Lys Ala
65                  70                  75                  80

Thr Asn Val Ala His Trp Thr Thr Pro Ser Leu Lys Cys Ile Arg Asp
                85                  90                  95

Pro Ala Leu Val His Gln Arg Pro Ala Pro Pro Ser Thr Val Thr Thr
            100                 105                 110

Ala Gly Val Thr Pro Gln Pro Glu Ser Leu Ser Pro Ser Gly Lys Glu
        115                 120                 125

Pro Ala Ala Ser Ser Pro Ser Ser Asn Asn Thr Ala Ala Thr Thr Ala
    130                 135                 140

Ala Ile Val Pro Gly Ser Gln Leu Met Pro Ser Lys Ser Pro Ser Thr
145                 150                 155                 160

Gly Thr Thr Glu Ile Ser Ser His Glu Ser Ser His Gly Thr Pro Ser
                165                 170                 175

Gln Thr Thr Ala Lys Asn Trp Glu Leu Thr Ala Ser Ala Ser His Gln
            180                 185                 190

Pro Pro Gly Val Tyr Pro Gln Gly His Ser Asp Thr Thr
        195                 200                 205

<210>  11
<211>  273
<212>  PRT
<213>  Artificial Sequence

<220>
<223>  Anti-CD24 scFv

<400>  11

Met Glu Phe Gly Leu Ser Trp Leu Phe Leu Val Ala Ile Leu Lys Gly
1               5                   10                  15

Val Gln Cys Asp Val His Leu Gln Glu Ser Gly Pro Asp Leu Val Lys
            20                  25                  30

Pro Ser Gln Ser Leu Ser Leu Thr Cys Thr Val Thr Gly Tyr Ser Ile
        35                  40                  45

Thr Ser Gly Tyr Thr Trp His Trp Ile Arg Gln Phe Pro Gly Asn Thr
    50                  55                  60

Val Glu Trp Met Glu Thr Gly Tyr Ile Gln Tyr Thr Gly Ser Thr Arg
65                  70                  75                  80

Tyr Asn Pro Ala Leu Arg Gly Arg Leu Ser Ile Ser Arg Asp Thr Ser
                85                  90                  95

Lys Asn Gln Phe Phe Leu Gln Leu Ile Ser Val Thr Thr Ala Asp Thr
            100                 105                 110

Gly Thr Tyr Phe Cys Ala Arg Gly Thr Thr Ala Ser Phe Asp Tyr Trp
        115                 120                 125

Gly Gln Gly Thr Thr Leu Thr Val Ala Ser Ala Gly Ser Ala Gly Gly
    130                 135                 140

Gly Gly Ser Gly Gly Gly Gly Ser Gly Gly Gly Gly Ser Asp Ile Val
145                 150                 155                 160

Met Ser Gln Ser Pro Ser Ser Leu Asn Val Ser Val Gly Glu Lys Val
                165                 170                 175

Thr Met Arg Cys Arg Ser Ser Gln Ser Leu Leu Tyr Ser Ser Asp Gln
            180                 185                 190

Lys Asn Tyr Leu Thr Trp Tyr Gln Gln Lys Pro Gly Gln Ser Pro Lys
        195                 200                 205

Leu Leu Ile Ser Trp Ala Ser Thr Arg Ala Ser Gly Val Pro Asp Arg
    210                 215                 220

Phe Thr Gly Ser Gly Ser Gly Thr Asp Phe Thr Leu Thr Ile Ser Ser
225                 230                 235                 240

Val Lys Ala Glu Asp Leu Gly Val Tyr Tyr Cys Gln Gln Tyr Phe Ile
                245                 250                 255

Tyr Pro Leu Thr Phe Gly Val Gly Thr Lys Leu Gly Leu Lys Ala Ala
            260                 265                 270

Ala

<210>  12
<211>  272
<212>  PRT
<213>  Artificial Sequence

<220>
<223>  Anti-CD56 scFv

<400>  12

Met Glu Phe Gly Leu Ser Trp Leu Phe Leu Val Ala Ile Leu Lys Gly
1               5                   10                  15

Val Gln Cys Ser Arg Gln Val Gln Leu Val Glu Ser Gly Gly Gly Val
            20                  25                  30

Val Gln Pro Gly Arg Ser Leu Arg Leu Ser Cys Ala Ala Ser Gly Phe
        35                  40                  45

Thr Phe Ser Ser Phe Gly Met His Trp Val Arg Gln Ala Pro Gly Lys
    50                  55                  60

Gly Leu Glu Trp Val Ala Tyr Ile Ser Ser Gly Ser Phe Thr Ile Tyr
65                  70                  75                  80

Tyr Ala Asp Ser Val Lys Gly Arg Phe Thr Ile Ser Arg Asp Asn Ser
                85                  90                  95

Lys Asn Thr Leu Tyr Leu Gln Met Asn Ser Leu Arg Ser Glu Asp Thr
            100                 105                 110

Ala Met Tyr Tyr Cys Ala Arg Met Arg Lys Gly Tyr Ala Met Asp Tyr
        115                 120                 125

Trp Gly Gln Gly Thr Leu Val Thr Val Ser Ser Gly Gly Gly Gly Ser
    130                 135                 140

Gly Gly Gly Gly Ser Gly Gly Gly Gly Ser Gly Gly Gly Gly Ser Asp
145                 150                 155                 160

Val Leu Met Thr Gln Thr Pro Leu Ser Leu Pro Val Thr Leu Gly Gln
                165                 170                 175

Pro Ala Ser Ile Ser Cys Arg Ser Ser Gln Ile Ile Ile His Ser Asp
            180                 185                 190

Gly Asn Thr Tyr Leu Glu Trp Phe Gln Gln Arg Pro Gly Gln Ser Pro
        195                 200                 205

Arg Leu Leu Ile Tyr Lys Val Ser Asn Arg Phe Ser Gly Val Pro Asp
    210                 215                 220

Arg Phe Ser Gly Ser Gly Ser Gly Thr Asp Phe Thr Leu Lys Ile Ser
225                 230                 235                 240

Arg Val Glu Ala Glu Asp Val Gly Val Tyr Tyr Cys Phe Gln Gly Ser
                245                 250                 255

His Val Pro His Thr Phe Gly Gly Gly Thr Lys Val Glu Ile Lys Pro
            260                 265                 270

<210>  13
<211>  285
<212>  PRT
<213>  Artificial Sequence

<220>
<223>  14g2a scFv

<400>  13

Met Glu Phe Gly Leu Ser Trp Leu Phe Leu Val Ala Ile Leu Lys Gly
1               5                   10                  15

Val Gln Cys Ser Arg Asp Ile Leu Leu Thr Gln Thr Pro Leu Ser Leu
            20                  25                  30

Pro Val Ser Leu Gly Asp Gln Ala Ser Ile Ser Cys Arg Ser Ser Gln
        35                  40                  45

Ser Leu Val His Arg Asn Gly Asn Thr Tyr Leu His Trp Tyr Leu Gln
    50                  55                  60

Lys Pro Gly Gln Ser Pro Lys Leu Leu Ile His Lys Val Ser Asn Arg
65                  70                  75                  80

Phe Ser Gly Val Pro Asp Arg Phe Ser Gly Ser Gly Ser Gly Thr Asp
                85                  90                  95

Phe Thr Leu Lys Ile Ser Arg Val Glu Ala Glu Asp Leu Gly Val Tyr
            100                 105                 110

Phe Cys Ser Gln Ser Thr His Val Pro Pro Leu Thr Phe Gly Ala Gly
        115                 120                 125

Thr Lys Leu Glu Leu Lys Arg Ala Asp Ala Ala Pro Thr Val Ser Ile
    130                 135                 140

Phe Pro Gly Ser Gly Gly Gly Gly Ser Gly Gly Glu Val Lys Leu Gln
145                 150                 155                 160

Gln Ser Gly Pro Ser Leu Val Glu Pro Gly Ala Ser Val Met Ile Ser
                165                 170                 175

Cys Lys Ala Ser Gly Ser Ser Phe Thr Gly Tyr Asn Met Asn Trp Val
            180                 185                 190

Arg Gln Asn Ile Gly Lys Ser Leu Glu Trp Ile Gly Ala Ile Asp Pro
        195                 200                 205

Tyr Tyr Gly Gly Thr Ser Tyr Asn Gln Lys Phe Lys Gly Arg Ala Thr
    210                 215                 220

Leu Thr Val Asp Lys Ser Ser Ser Thr Ala Tyr Met His Leu Lys Ser
225                 230                 235                 240

Leu Thr Ser Glu Asp Ser Ala Val Tyr Tyr Cys Val Ser Gly Met Glu
                245                 250                 255

Tyr Trp Gly Gln Gly Thr Ser Val Thr Val Ser Ser Ala Lys Thr Thr
            260                 265                 270

Pro Pro Ser Val Tyr Gly Arg Val Thr Val Ser Ser Ala
        275                 280                 285

<210> 14
<211> 20
<212> PRT
<213> equine rhinitis A virus

<400> 14

Gln Cys Thr Asn Tyr Ala Leu Leu Lys Leu Ala Gly Asp Val Glu Ser
1               5                   10                  15

Asn Pro Gly Pro
            20

<210> 15
<211> 5
<212> PRT
<213> Artificial Sequence

<220>
<223> linker sequence

<400> 15

Gly Gly Gly Gly Ser
1               5

## Description

### TECHNICAL FIELD

Embodiments of the disclosure are related to at least the fields of cell biology, molecular biology, immunology, and medicine.

### BACKGROUND

Although immunotherapy of B-cell malignancies with T cells expressing CD19-specific chimeric antigen receptors (CAR19) has produced high rate of complete responses, immunotherapy of solid tumors with CARs or engineered T cell receptors (TCRs) that recognize other antigens has benefited only a few patients (1, 2). It is still unknown whether CAR19 and/or its interaction with CD19 antigen have unique properties that contribute to the efficacy of CAR T cell therapy in B-cell malignancies.

Among the major known factors that negatively affect susceptibility of solid tumors to adoptive immunotherapy, including CAR- and TCR-redirected immunotherapy are poor localization of therapeutic effector cells to the tumor site and immunosuppressive tumor microenvironment (TME) (3). Neuroblastoma (NB) is a typical solid tumor which does not express CD19 and has a highly immunosuppressive TME. Early clinical studies with CAR-redirected T cells in NB patients have targeted a GD2 ganglioside, which is overexpressed in NB cells (4, 5). The immunotherapy with CARGD2 T cells in NB patients was well tolerated and produced a number of objective clinical responses but the rate of such responses was much lower compared to the immunotherapy with CAR19 T cells in B-cell malignancies (6).

Va24-invariant NKT and CARGD2 NKT cells better localize to neuroblastoma xenografts in mice compared to T and CARGD2 T cells (7). Unlike T cells, NKT cells can kill or reprogram immunosuppressive tumor-associated macrophages (8). Therefore, better localization to the tumor site and neutralization of suppressive TAMs give NKTs an advantage over T cells as the effector population for CAR-redirected immunotherapy of solid tumors.

Despite effective localization to the tumor site, the functionality and therapeutic efficacy of NKT cells are inhibited by hypoxic TME. NKT cells can be rescued from hypoxia-mediated suppression by IL-15 so that NKT cells engineered to express IL-15 showed increased anti-tumor activity in a NB model in mice (9). Furthermore, NKT cells that co-express a CARGD2 and IL-15 in the same construct (CARGD2.IL15) undergo *in vivo* expansion in NB-bearing mice and have a higher anti-tumor activity compared with NKTs expressing either CARGD2 or IL-15 alone. In addition, NK cell proliferation, survival, and antibody-mediated cytotoxicity can be increased in the presence of a chimeric protein of the sushi domain of IL15Ra, IL-15, and apolipoprotein (10).

The present disclosure provides solutions to long-felt needs in the art of adoptive immunotherapy for the treatment of cancer.

### BRIEF SUMMARY

The present disclosure is directed to systems, compositions, and methods that expand the use of engineered immune cells having a receptor that targets an antigen. In particular embodiments, the disclosure concerns cancer immunotherapy and configuring the immunotherapy with modular molecular adaptors that allow for interchangeable use depending upon the cancer to be treated and its particular expression of tumor antigen(s). In some embodiments, the disclosure concerns cancer immunotherapy and molecular adaptors that at least in some cases allow for utilizing pre-existing immune effector cells that target a first antigen to also target a second (or more) antigen.

Embodiments of the disclosure include the engineering of immune effector cells to express an engineered antigen receptor and to secrete a molecular adaptor with an antigen-cytokine complex for effective cancer immunotherapy. In particular embodiments, the molecular adaptor in comprising a cytokine domain (cytokine, a cytokine receptor, and/or a cytokine and its receptor) allows for use of immunotherapy in suppressive microenvironments of solid tumors.

In a certain embodiment, there is an immune effector cell, wherein the cell produces a molecular adaptor comprising: (a) a tumor cell binding domain, and (b) a cytokine domain comprising (i) a cytokine or a functional fragment thereof; or (ii) a cytokine receptor or a functional fragment thereof; or (iii) a combination of both (i) and (ii); (c) optionally further comprising an antigen receptor target domain; optionally wherein the cell comprises one or more engineered antigen receptors against one or more tumor target antigens. Thus, the immune effector cell is engineered and therefore non-natural.

In a certain embodiment, there is an immune effector cell, wherein the cell produces a molecular adaptor comprising: (a) a target cell binding domain, and (b) a cytokine domain comprising (i) a cytokine or a functional fragment thereof; or (ii) a cytokine receptor or a functional fragment thereof; or (iii) a combination of both (i) and (ii); (c) optionally further comprising an antigen receptor target domain; optionally wherein the cell comprises one or more engineered antigen receptors against one or more target antigens. Thus, the immune effector cell is engineered and therefore non-natural.

In one embodiment, a molecular adaptor is provided that comprises a) a tumor cell binding domain, (b) a cytokine domain comprising (i) a cytokine or a functional fragment thereof; or (ii) a cytokine receptor or a functional fragment thereof; or (iii) a combination of both (i) and (ii), and optionally (c) an antigen receptor target domain. Such molecular adaptor can be provided in combination with engineered immune effector cells comprising one or more engineered antigen receptors against one or more tumor target antigens to improve efficacy of the immunotherapy.

Embodiments of the disclosure include an immune effector cell, wherein the cell produces a molecular adaptor comprising: (a) a tumor cell binding domain, and (b) a cytokine domain comprising (i) a cytokine or a functional fragment thereof; or (ii) a cytokine receptor or a functional fragment thereof; or (iii) a combination of both (i) and (ii); (c) optionally (or not) further comprising an antigen receptor target domain; optionally (or not) wherein the cell comprises one or more engineered antigen receptors against one or more tumor target antigens. In specific embodiments, the tumor cell binding domain comprises an antibody, an antibody fragment or a non-antibody scaffold. The tumor cell binding domain may comprise an scFv. The tumor cell binding domain may target an antigen expressed on the surface of a tumor cell, such as GD2, CD56, CD24, LICAM, GPC2, GPC3, CSPG4, HER2, CD19, CD22, CD30, CD71, B7-H3, B7-H4, BCMA, PD-L1, or PDL-2. In some cases, the cytokine is selected from the group consisting of IL-7, IL-12, IL-15, IL-18, IL-27, IL-33, or IL-21. In specific cases, the cytokine is IL-15 and/or the cytokine receptor is IL-15R or the alpha subunit thereof (IL15RA). The antigen receptor target domain may comprise a tumor antigen or a functional fragment thereof, or the antigen receptor target domain could also be an antigen (or a functional fragment thereof) that is not considered a tumor antigen.

In particular embodiments, the molecular adaptor comprises a tumor cell binding domain targeting a first antigen on the tumor cell and an antigen receptor target domain comprising a second tumor antigen or functional fragment thereof. In specific embodiments, the antigen receptor target domain comprises CD19. Any cell of the disclosure may comprise one or more engineered antigen receptors that are an exogenously provided T cell receptor (TCR) or a chimeric antigen receptor (CAR). The cell may comprise an engineered TCR by virtue of transduction with a vector that encodes a TCR, including encodes a TCR from a natural source.

In particular embodiments, a cell of the disclosure produces a molecular adaptor targeting a first tumor antigen and comprises an antigen receptor targeting a first tumor antigen; produces a molecular adaptor targeting a second tumor antigen and comprises an antigen receptor targeting a first tumor antigen; or produces a molecular adaptor targeting a first tumor antigen, said molecular adaptor comprising an antigen receptor target domain that is targeted by an antigen receptor expressed on the surface of the cell. In specific embodiments, the tumor cell binding domain and the antigen receptor target domain are linked by a hinge region and/or linker. The hinge region may be from IgG1, IgG2, IgG3, IgG4, or CD8. In specific embodiments, the cell comprises an expression vector (viral vector or a non-viral vector) or RNA encoding said molecular adaptor and optionally said engineered antigen receptor. Examples of viral vectors include retroviral, lentiviral, adenoviral, or adeno-associated viral vector. Examples of non-viral vectors include plasmids and transposons. In specific embodiments, the cell is an NK cell, an NKT cell, a T cell, a γδ T cell, a Mucosal-associated invariant T (MAIT) cell, an innate lymphoid (IL) cell, cytokine-induced killer (CIK) cell, macrophage, a stem cell, or a mixture thereof.

In one embodiment, there is a molecular adaptor for redirecting cells expressing engineered antigen receptors, comprising: (a) a tumor cell binding domain, (b) a cytokine domain comprising (i) a cytokine or a functional fragment thereof; or (ii) a cytokine receptor or a functional fragment thereof; or (iii) a combination of both (i) and (ii), and (c) an antigen receptor target domain. In specific embodiments, the cytokine domain in the molecular adaptor is positioned between the tumor cell binding domain and the antigen receptor domain.

In one embodiment, there is a molecular adaptor for redirecting cells expressing engineered antigen receptors, comprising: (a) a target cell binding domain, (b) a cytokine domain comprising (i) a cytokine or a functional fragment thereof; or (ii) a cytokine receptor or a functional fragment thereof; or (iii) a combination of both (i) and (ii), and (c) an antigen receptor target domain. In specific embodiments, the cytokine domain in the molecular adaptor is positioned between the target cell binding domain and the antigen receptor domain.

Embodiments of the disclosure include non-natural antigen receptors directed against a molecular adaptor encompassed by the disclosure.

In one embodiment, there is an expression vector encoding a molecular adaptor of the disclosure. In some cases the expression vector comprises sequence that encodes an antigen receptor. Particular embodiments include an expression vector encoding: a molecular adaptor comprising: (i) a tumor cell binding domain, and (ii) a cytokine domain; and (b) an antigen receptor. In a specific embodiments, an IRES element or a 2A sequence is located between the molecular adaptor and the antigen receptor. Such an expression vector may be a viral vector (retroviral, lentiviral, adenoviral, or adeno-associated viral vector) or a non-viral vector.

In one embodiment, there is an expression vector encoding a molecular adaptor of the disclosure. In some cases the expression vector comprises sequence that encodes an antigen receptor. Particular embodiments include an expression vector encoding: a molecular adaptor comprising: (i) a target cell binding domain, and (ii) a cytokine domain; and (b) an antigen receptor. In a specific embodiments, an IRES element or a 2A sequence is located between the molecular adaptor and the antigen receptor. Such an expression vector may be a viral vector (retroviral, lentiviral, adenoviral, or adeno-associated viral vector) or a non-viral vector.

Embodiments of the disclosure encompass cells comprising an expression vector encompassed by the disclosure.

In one embodiment, there is a method of treating cancer or other disease (autoimmune disease, for example) in an individual, comprising the step of administering to the individual a therapeutically effective amount of the cells of the disclosure. In cases wherein the disease is not cancer, the disease may have conditions where it is desirable to kill a population of cells that can be targeted through one or more antigens associated with the disease.

In a particular embodiment, there is a method of treating cancer or other disease in an individual, comprising administering a therapeutically effective amount of a molecular adaptor of the disclosure and/or a therapeutically effective amount of cells expressing an antigen receptor of the disclosure.

In a certain embodiment, there is a method of treating cancer in an individual, comprising administering to the individual a therapeutically effective amount of: a molecular adaptor comprising a tumor cell binding domain targeting a first or a second antigen on a tumor cell and a cytokine domain, and cells expressing an antigen receptor against a first antigen on the tumor cell. In specific embodiments, the molecular adaptor and/or the cells are administered to the individual by intraperitoneal or intravenous injection. In some cases, a second dose of the molecular adaptor is administered after the initial dose. The individual may be receiving, has received, and/or will receive an additional cancer therapy. An additional cancer therapy may be surgery, radiation, chemotherapy, immunotherapy, hormone therapy, or a combination thereof.

In a certain embodiment, there is a method of treating a disease in an individual, comprising administering to the individual a therapeutically effective amount of: a molecular adaptor comprising a target cell binding domain targeting a first or a second antigen on a target cell and a cytokine domain, and cells expressing an antigen receptor against a first antigen on the target cell. In specific embodiments, the molecular adaptor and/or the cells are administered to the individual by intraperitoneal or intravenous injection. In some cases, a second dose of the molecular adaptor is administered after the initial dose. The individual may be receiving, has received, and/or will receive an additional therapy for the disease.

In some embodiments, there is a pharmaceutical composition comprising immune effector cells encompassed herein and/or a molecular adaptor encompassed herein.

In one embodiment, there is a method of adapting a cancer therapy to a cancer expressing a first tumor antigen, comprising the steps of: (a) providing or obtaining an immune effector cell or plurality thereof comprising a non-natural antigen receptor that targets a second tumor antigen; and (b) providing or obtaining a molecular adaptor comprising a tumor cell binding domain that binds the first tumor antigen and an antigen receptor target domain that binds the antigen receptor that targets a second tumor antigen, optionally (or not) wherein the molecular adaptor comprises (i) a cytokine or a functional fragment thereof; or (ii) a cytokine receptor or a functional fragment thereof; or (iii) a combination of both (i) and (ii), and wherein the providing or obtaining of the molecular adaptor in step (b) is determined by the presence of expression of the first antigen in the cancer cells of the cancer being treated.

In a certain embodiment, there is a method of adapting a cancer therapy to a cancer expressing a first tumor antigen, comprising the steps of: (a) providing or obtaining an immune effector cell or plurality thereof comprising a non-natural antigen receptor that targets a second tumor antigen; and (b) transfecting said cells with a polynucleotide that encodes a molecular adaptor comprising: (1) a tumor cell binding domain that targets the first antigen, and (2) a cytokine domain comprising: (i) a cytokine or a functional fragment thereof; or (ii) a cytokine receptor or a functional fragment thereof; or (iii) a combination of both (i) and (ii).

In one embodiment, there is a method of adapting a disease therapy to a diseased cell expressing a first target cell antigen (that may also be the diseased cell antigen), comprising the steps of: (a) providing or obtaining an immune effector cell or plurality thereof comprising a non-natural antigen receptor that targets a second antigen; and (b) providing or obtaining a molecular adaptor comprising a target cell binding domain that binds the first antigen and an antigen receptor target domain that binds the antigen receptor that targets a second antigen, optionally (or not) wherein the molecular adaptor comprises (i) a cytokine or a functional fragment thereof; or (ii) a cytokine receptor or a functional fragment thereof; or (iii) a combination of both (i) and (ii), and wherein the providing or obtaining of the molecular adaptor in step (b) is determined by the presence of expression of the first antigen in the target cells of the disease being treated.

In a certain embodiment, there is a method of adapting a disease therapy to a cancer expressing a first antigen, comprising the steps of: (a) providing or obtaining an immune effector cell or plurality thereof comprising a non-natural antigen receptor that targets a second antigen; and (b) transfecting said cells with a polynucleotide that encodes a molecular adaptor comprising: (1) a target cell binding domain that targets the first antigen, and (2) a cytokine domain comprising: (i) a cytokine or a functional fragment thereof; or (ii) a cytokine receptor or a functional fragment thereof; or (iii) a combination of both (i) and (ii).

The foregoing has outlined rather broadly the features and technical advantages of the present invention in order that the detailed description of the invention that follows may be better understood. Additional features and advantages of the invention will be described hereinafter which form the subject of the claims of the invention. It should be appreciated by those skilled in the art that the conception and specific embodiment disclosed may be readily utilized as a basis for modifying or designing other structures for carrying out the same purposes of the present invention. It should also be realized by those skilled in the art that such equivalent constructions do not depart from the spirit and scope of the invention as set forth in the appended claims. The novel features which are believed to be characteristic of the invention, both as to its organization and method of operation, together with further objects and advantages will be better understood from the following description when considered in connection with the accompanying figures. It is to be expressly understood, however, that each of the figures is provided for the purpose of illustration and description only and is not intended as a definition of the limits of the present invention.

### BRIEF DESCRIPTION OF THE DRAWINGS

For a more complete understanding of the present disclosure, reference is now made to the following descriptions taken in conjunction with the accompanying drawing, in which:

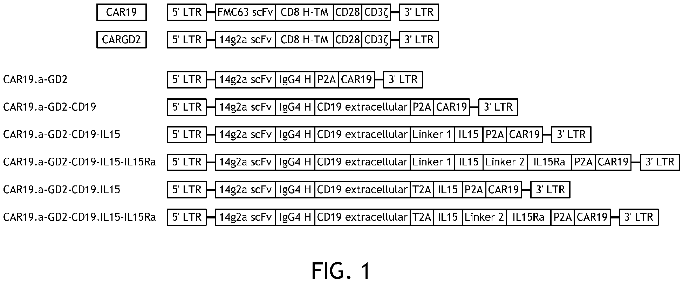

**FIG. 1****. Schematic Diagram of Molecular Adaptor Constructs:** "FMC63 scFv" is an anti-CD 19 scFv domain, "CD8 H-TM" is the human CD8 hinge region and CD8 transmembrane domain, "CD28" is the costimulatory domain from CD28, "CD3ζ" is the CD3ζ T-cell activation domain, "14g2a scFv" is an anti-GD2 scFv domain, "IgG4 H" is the hinge region from human IgG4, "P2A" is the self-cleaving viral P2A sequence, "CAR19" is the full length CD 19 CAR construct shown at the top of the Figure, "CD 19 extracellular" is the human CD19 extracellular domain or antigen for the CD19 CAR, "Linker 1" is a 20 amino acid linker, "IL15" is human IL-15, "Linker 2" is a 26 amino acid linker, "IL15Ra" is the human IL-15 receptor alpha subunit, "T2A" is the self-cleaving viral T2A sequence, "5' LTR is the 5' long terminal repeat; and "3' LTR" is the 3' long terminal repeat;

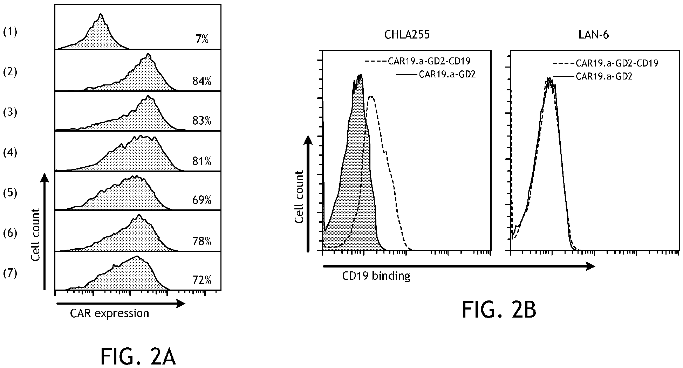

**FIGS. 2A and 2B****. Expression and Specificity of a-GD2-CD19 Molecular Adaptor with or without Cytokine Complex: (2A)** Expression of CAR19 by transduced NKT cells was determined by flow cytometry staining with anti-CAR19 antibody. Therein, (1) refers to Non-transduced; (2) refers to CAR19.a-GD2; (3) refers to CAR19.a-GD2-CD19; (4) refers to CAR19.a-GD2-CD19-IL15; (5) refers to CAR19.a-GD2-CD19-IL15-IL15Ra; (6) refers to CAR19.a-GD2-CD19.IL15; and (7) refers to CAR19.a-GD2-CD19.IL15-IL15Ra; percentages represent the percentage of NKT cells that express CAR19; (**2B**) Specificity of the molecular adaptors was determined by incubating supernatants from CAR19.a-GD2-CD19 or CAR19.a-GD2 transduced NKT cells with CD19-negative/GD2-positive CHLA-255, or CD19-negative/GD2-negative LAN-6 neuroblastoma cells. The binding of a-GD2-CD19 to neuroblastoma cells was analyzed by flow cytometry after staining with anti-CD 19 antibody. Results from a representative of three experiments;

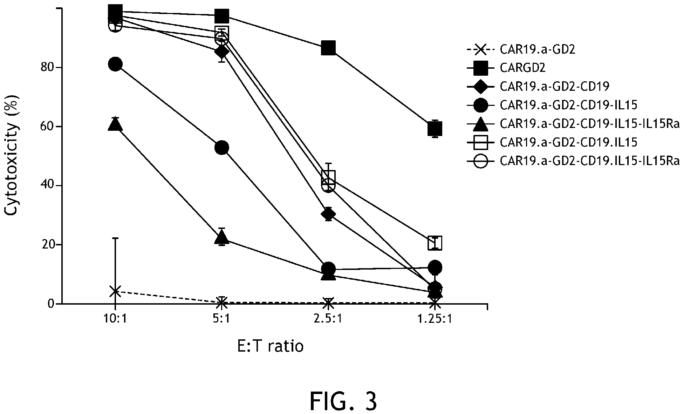

**FIG. 3****. Molecular Adaptors Mediate Effective Killing of NB Cells in vitro:** Cell mediated cytotoxicity was analyzed after incubating effector NKT cells transduced with the indicated constructs and target CHLA-255 NB cells stably expressing luciferase. Luciferase activities (live cells) were measured after co-culturing for 4 hours at varying effector to target (E:T) ratios. Results are plotted as percent cytotoxicity from a representative of four experiments;

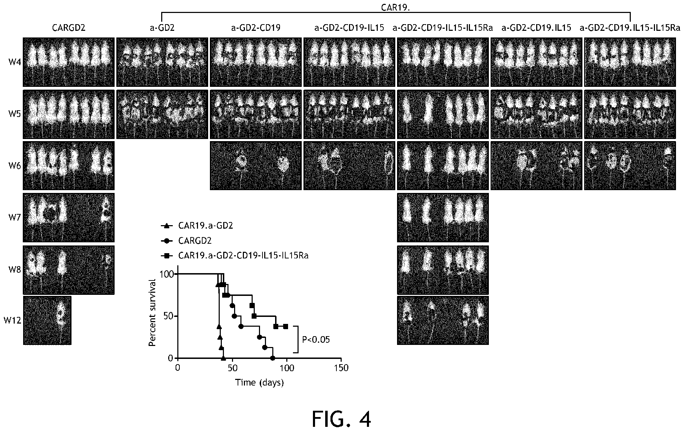

**FIG. 4****. Molecular Adaptor with IL-15/IL-15R Complex Mediates Potent Antitumor Activity against NB Tumor Xenografts in NSG mice.** The antitumor activity of NKT cells transduced with the indicated constructs was measured in a metastatic neuroblastoma model. 10⁶ CHLA255/Luc NB cells were injected into NSG mice followed 1 week later by injection of 10⁷ of the transduced NKT cells. Tumor growth was monitored weekly from week 4 after tumor injection using bioluminescent imaging. Survival probability was analyzed using Kaplan-Meier method. Results from a representative of two experiments;

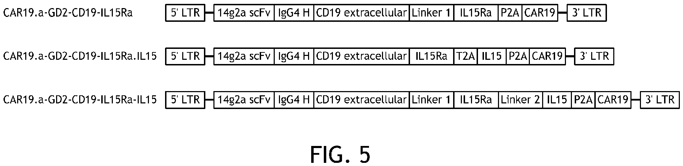

**FIG. 5****. Schematic Diagram of Additional Molecular Adaptor and Cytokine Constructs:** "14g2a scFv" is an anti-GD2 scFv domain, "IgG4 H" is the hinge region from IgG4, "P2A" is the self-cleaving viral P2A sequence, "CAR19" is the full length anti-CD 19 CAR construct shown at the top of FIG. 1, "CD19 extracellular" is the human CD19 extracellular domain or antigen for the anti-CD 19 CAR, "Linker 1" is a 20 amino acid linker, "IL15" is human IL15, "Linker 2" is a 26 amino acid linker, "IL15Ra" is the human IL15Ra, "T2A" is the self-cleaving viral T2A sequence, "5' LTR" is a 5' long terminal repeat, and "3' LTR" is a 3' long terminal repeat;

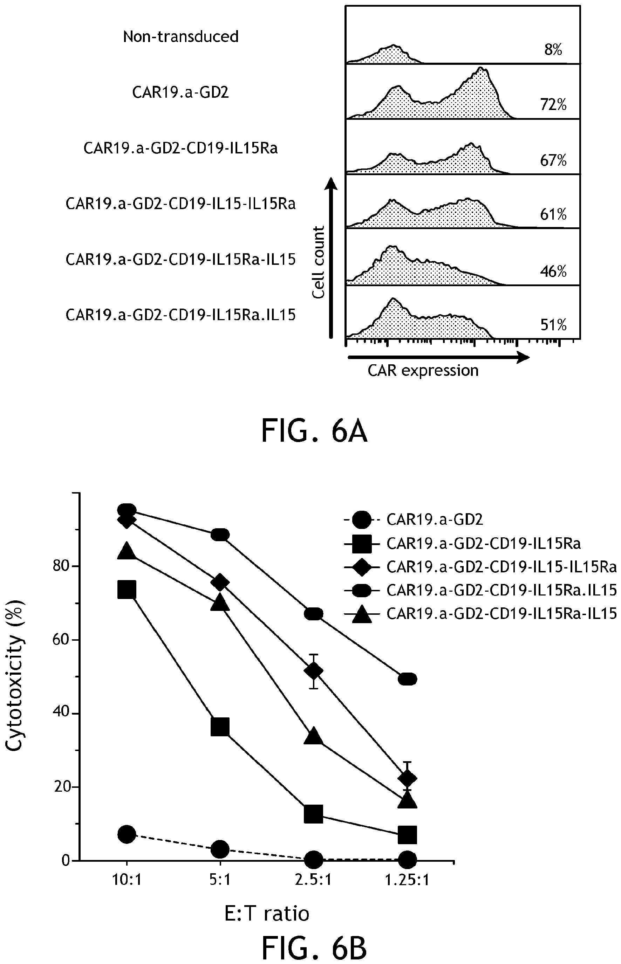

**FIGS. 6A and 6B****. Expression of and *in vitro* Cytotoxicity Mediated by Additional GD2 Molecular Adaptors: (6A)** *In vitro* stimulated NKT cells were transduced with the indicated molecular adaptor constructs. CAR expression was analyzed by flow cytometry after staining with anti-CD 19 CAR antibody. Percentages represent NKT cells expressing CAR19. (**6B**) Cell mediated cytotoxicity was analyzed by incubating effector NKT cells transduced with the indicated constructs with target neuroblastoma cells at various effector to target cell ratios. Results from a representative of two experiments;

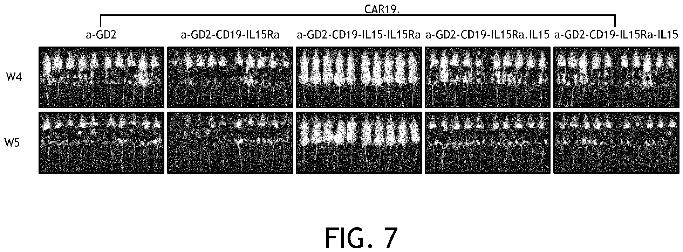

**FIG. 7****. Molecular Adaptors with a defined IL-15/IL-15R Complex Orientation Mediates Potent Antitumor Activity against NB Tumor Xenografts in NSG mice.** The antitumor activity of NKT cells transduced with the indicated constructs was measured in a metastatic neuroblastoma model as described in FIG. 4. Results from a representative of two experiments;

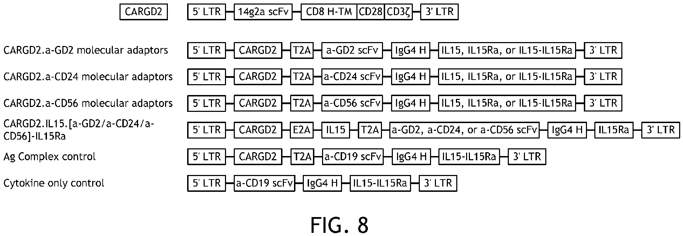

**FIG. 8****. Schematic presentation of retroviral constructs encoding CARGD2 and antigen-cytokine complexes of IL15/IL15Ra linked to scFv against GD2, CD24, or CD56 on neuroblastoma cell surface.** Schematically describes the constructs designed to present the IL15/IL15Ra on neuroblastoma through targeting with GD2, CD24, or CD56. CARGD2 expressing cells with or without transgenic expression of IL15, co-expressing a molecular adaptor with anti-GD2 (a-GD2 scFv), anti-CD24 (a-CD24 scFv), or anti-CD56 (a-CD56 scFv) fused to IL15, IL15Ra, or both. Two controls included: i) CARGD2 with IL15-IL15Ra complex targeting CD19 Ag, not expressed in neuroblastoma; ii) no CAR with IL15-IL15Ra complex targeting CD19; "E2A" is the self-cleaving viral E2A sequence; other labels are as described in FIG. 1 and FIG. 5;

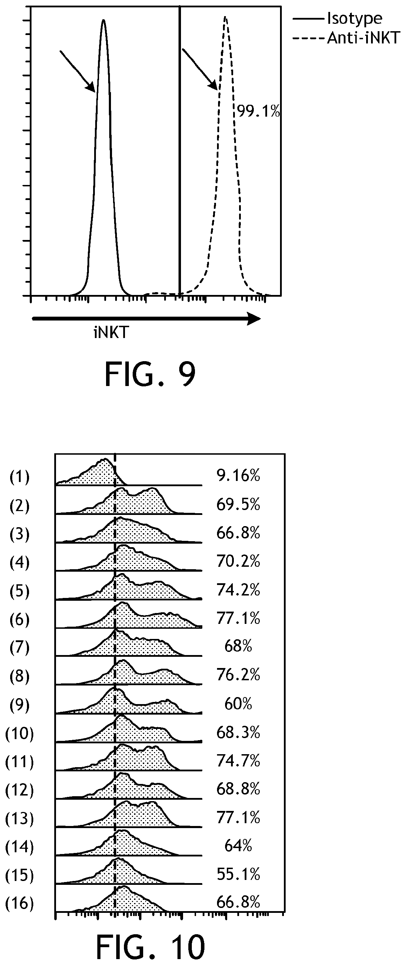

**FIG. 9****. NKT purity check.** NKT cell purity was determined by flow cytometry in which the cells were stained using BV421 Mouse Anti-Human Invariant NKT Cell antibody specific for the invariant iNKT TCR Vα24Jα18 (BD Bioscience, clone 6B11), and BV421 Mouse IgG1, k Isotype Control antibody (Bioscience, clone X40), separately;
**FIG. 10****. Constructs encoding CARGD2 and different antigen-cytokine complexes allowed efficient CAR expression in NKT cells.** CAR expression levels in transduced NKT cells were determined by anti-mouse F(ab')₂ antibody using flow cytometry (Alexa Fluor® 647 AffiniPure F(ab')₂ Fragment Goat Anti-Mouse IgG, F(ab')₂ fragment specific, Cat No: 115-606-006). In FIG. 10, (1) refers to Non-transduced NKT cells; (2) refers to CARGD2; (3) refers to CARGD2.IL15; (4) refers to CARGD2.IL15-IL15Ra; (5) refers CARGD2.a-GD2-IL15; (6) refers to CARGD2.a-GD2-IL15Ra; (7) refers to CARGD2.a-GD2-IL15-IL15Ra; (8) refers to CARGD2.a-CD24-IL15; (9) refers to CARGD2.a-CD24-IL15Ra; (10) refers to CARGD2.a-CD24-IL15-IL15Ra; (11) refers to CARGD2.a-CD56-IL15; (12) refers to CARGD2.a-CD56-IL15Ra; (13) refers to CARGD2.a-CD56-IL15-IL15Ra; (14) refers to CARGD2.IL15.a-GD2-IL15Ra; (15) refers to CARGD2.IL15.a-CD24-IL15Ra; and (16) refers to CARGD2.IL15.a-CD56- IL15Ra;

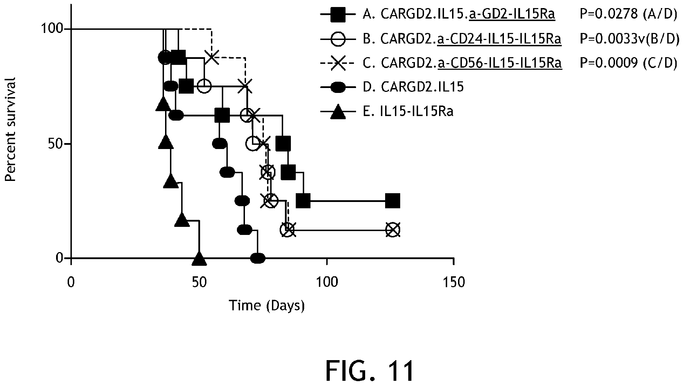

FIG. 11. Log-rank (Mantel-Cox) test showing significantly enhanced anti-tumor activity through IL15Ra or IL-15-IL15Ra molecular adaptor compared to IL-15-secreting CARGD2-cells. Survival plot is generated using percentage of survival of mouse on days in groups treated with CARGD2 iNKT cells expressing different adaptors. Data were analyzed by the Kaplan Meier method. The differences in survival were then compared using the Log-rank test. P values are group A, B, or C comparing with group D;

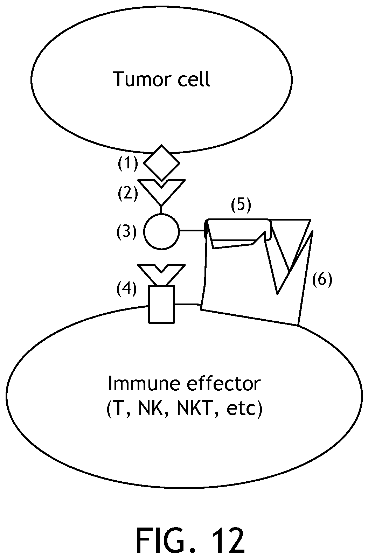

FIG. 12. Example of a Model of a CAR Molecular Adaptor with an Antigen-**Cytokine Complex.** One exemplary molecular adaptor comprises a fusion of CD19 extracellular domain and a scFv derived from a mAb specific for GD2 (a-GD2-CD19). NKT cells transduced with a construct containing CAR19 and an a-GD2-CD19adaptor express CAR19 on the cell surface and secrete such molecular adaptor. The antigen part of the molecular adaptor (*e.g*., CD19 extracellular) is fused with an activating cytokine domain (*e.g.,* IL15-IL15Ra), thereby forming an antigen-cytokine complex on the tumor cell surface that is simultaneously recognized by the CAR and the respective cytokine receptor expressed by an immune effector cell. In FIG. 12, (1) refers to GD2; (2) refers to anti-GD2 scFv (a-GD2); (3) refers to the CD19 extracellular domain; (4) refers to CAR19; (5) refers to IL15-IL15Ra; and (6) refers to IL-2Rβ/γ. Note that (2), (3), and (5) are connected by amino acid linkers (blue lines); and

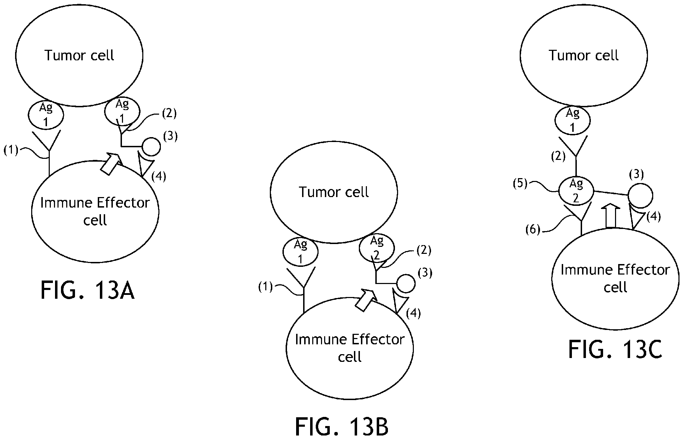

**FIGS. 13A-13C****. Embodiments of Immune Effector Cell Modifications and Interactions with Tumor Cells.** (**13A**) An immune effector cell produces and secretes a molecular adaptor targeting a first tumor antigen (Ag 1) and comprises an antigen receptor targeting a first tumor antigen. (**13B**) An immune effector cell produces a molecular adaptor targeting a second tumor (Ag 2) antigen and comprises an antigen receptor targeting a first tumor antigen. (**13C**) An immune effector cell produces a molecular adaptor targeting a first tumor antigen, said molecular adaptor having an antigen receptor target domain that is targeted by an antigen receptor expressed on the surface of the cell. In FIG. 13, (1) refers to antigen receptor (TCR, CAR, etc.) against a first tumor target antigen; (2) refers to tumor cell binding domain; (3) refers to cytokine domain; (4) refers to cytokine receptor; (5) refers to antigen receptor target domain; and (6) refers to an antigen receptor against a second tumor target antigen. In specific cases, as exemplified in FIG. 13C, element (6) is not a TCR because the antigen is soluble.

### DETAILED DESCRIPTION

As used herein the specification, "a" or "an" may mean one or more. As used herein in the claim(s), when used in conjunction with the word "comprising", the words "a" or "an" may mean one or more than one. As used herein "another" may mean at least a second or more. In specific embodiments, aspects of the invention may "consist essentially of' or "consist of' one or more sequences of the invention, for example. Some embodiments of the invention may consist of or consist essentially of one or more elements, method steps, and/or methods of the invention. It is contemplated that any method or composition described herein can be implemented with respect to any other method or composition described herein. The scope of the present application is not intended to be limited to the particular embodiments of the process, machine, manufacture, composition of matter, means, methods and steps described in the specification.

In certain embodiments, the present disclosure concerns immune effector cells, preferably NKT cells, engineered to express an antigen receptor, such as a CAR, against a first tumor antigen that can be effectively redirected against tumor cells expressing a second antigen using a certain molecular adaptor approach described herein. In at least specific cases, an IL-15-IL-15R complex or a similar pro-survival cytokine-cytokine receptor complex is co-presented with the molecular adaptor-delivered target antigen on the tumor cell surface. This allows antigen receptor-expressing immune effector cells to recognize a target antigen and a cytokine in the close molecular proximity on the tumor cell surface.

In other embodiments, the present disclosure concerns immune effector cells, in preferably NKT cells, engineered to express an antigen receptor, such as a CAR, against a first tumor antigen, wherein said cell co-expresses a molecular adaptor that comprises a tumor cell binding domain and a cytokine domain. *Via* the tumor cell binding domain, the molecular adaptor may bind to the tumor cell and the immune effector cell may in turn bind *e.g. via* cytokine receptors to the molecular adaptor, thereby combining activating and survival signals in the same immune effector cell that maximizes its anti-tumor activity.

In some embodiments, the present disclosure concerns immune effector cells, preferably NKT cells, engineered to express an antigen receptor, such as a CAR, against a first antigen that can be effectively redirected against target cells expressing a second antigen using a certain molecular adaptor approach described herein. In at least specific cases, an IL-15-IL-15R complex or a similar pro-survival cytokine-cytokine receptor complex is co-presented with the molecular adaptor-delivered target antigen on the cell surface. This allows antigen receptor-expressing immune effector cells to recognize a target antigen and a cytokine in the close molecular proximity on the target cell surface.

As shown herein, the concepts of engineering immune effector cells to express a tumor-specific antigen receptor and to deliver a target tumor antigen with a fused cytokine are novel, effective in an *in vivo* model of neuroblastoma, and broadly applicable to immunotherapy of all cancers.

### I. Cells and Related Components and Compositions

Embodiments of the disclosure encompass cells that are not found in nature and are manipulated by the hand of man. Thus, the cells are engineered (or synthetic) and therefore non-natural. The cells may be isolated cells. The cells comprise at least one entity, such as a nucleic acid entity or proteinaceous entity, that is recombinant, exogenously provided to the cell, synthetic, and/or transgenic, and so forth.

Cells encompassed herein include immune effector cells of any kind. Such cells may be NK cell, an NKT cell, a T cell, a γδ T cell, a Mucosal-associated invariant T (MAIT) cell, an innate lymphoid (IL) cell, cytokine-induced killer (CIK) cell, a macrophage, a stem cell, a mixture thereof, and so forth, in certain embodiments. NKT cells are in preferred embodiments CD1d restricted and preferably invariant NKTs (iNKTs; also termed type 1 NKTs) (11), such as Vα24i NKT or Vα24-Jα18 NKTs). The immune effector cells in particular embodiments are engineered to produce, including secrete in at least some cases, one or more compositions that are non-natural to the cell. In additional or alternative embodiments the cells are engineered to express one or more compositions that are not secreted from the cell. Preferably, the immune effector cells are human.

Particular embodiments include immune effector cells that produce one or more soluble fusion proteins herein referred to as "molecular adaptor" (see FIG. 13). Such molecular adaptors in specific embodiments comprise (a) a tumor cell binding domain; (b) a cytokine domain comprising (i) a cytokine or a functional fragment thereof; or (ii) a cytokine receptor or a functional fragment thereof; or (iii) a combination of both (i) and (ii); and(c) optionally further comprising an antigen receptor target domain.

In particular embodiments, the molecular adaptors comprise at least one tumor cell binding domain that targets an antigen expressed on the surface of a tumor cell (or a non-solid cancer cell such as a cancer cell of a hematological malignancy). In cases wherein the immune effector cells produce molecular adaptors that comprise a tumor cell binding domain, the tumor cell binding domain may be of any suitable kind. In specific embodiments, the tumor cell binding domain comprises an antibody of any kind (including functional fragments thereof) and/or a non-antibody scaffold. The tumor cell binding domain that comprises an antibody may comprise a full-length antibody. In some cases the antibody is a chimeric antibody. In cases wherein the tumor cell binding domain is a functional antibody fragment, the antibody fragment may be a Fab; a Fab'; a F(ab)'₂; a scFv; a Fv fragment; a scFab; a nanobody; a VHH or a minimal recognition unit; bispecific or multispecific molecules such as a diabody, a single-chain diabody, a DART, a BiTE, a tandem scFv; or a combination thereof. In cases wherein the tumor cell binding domain comprises a non-antibody scaffold, the non-antibody scaffold may comprise an affibody, an affilin molecule, an AdNectin, a lipocalin mutein, a DARPin, a Knottin, a Kunitz-type domain, an Avimer, a Tetranectin, a trans-body, Fibronectin type II domains, a soluble receptor or ligand for the molecule being target on the tumor, or a combination thereof. In particular embodiments, the tumor cell binding domain is an antibody fragment that is a scFv. Such an scFv may be targeted against any tumor cell antigen. Examples of tumor cell antigens to which the scFv may be directed include at least 5T4, 8H9, αᵥβ₆ integrin, 5T4, 8H9, αᵥβ₆ integrin, BCMA, B7-H3, B7-H6, CAIX, CA9, CD19, CD20, CD22, CD30, CD33, CD38, CD44, CD44v6, CD44v7/8, CD70, CD123, CD138, CD171, CEA, CSPG4, EGFR, EGFR family including ErbB2 (HER2), EGFRvIII, EGP2, EGP40, ERBB3, ERBB4, ErbB3/4, EPCAM, EphA2, EpCAM, folate receptor-a, FAP, FBP, fetal AchR, FRα, GD2, G250/CAIX, GD3, Glypican-3 (GPC3), Her2, HLA-A1+MAGE1, HLA-A1+NY-ESO-1, IL-11Rα, IL-13Rα2, Lambda, Lewis-Y, Kappa, KDR, Melanoma-associated antigen (MAGE), MCSP, Mesothelin, Muc1, Muc16, NCAM, NKG2D Ligands, NY-ESO-1, Preferentially expressed antigen of melanoma (PRAME), PSC1, PSCA, PSMA, ROR1, SP17, Survivin, TAG72, TEMs, carcinoembryonic antigen, HMW-MAA, AFP, CA-125, ETA, Tyrosinase, MAGE, laminin receptor, HPV E6, E7, BING-4, Calcium-activated chloride channel 2, Cyclin-Bl, 9D7, EphA3, Telomerase, SAP-1, BAGE family, CAGE family, GAGE family, MAGE family, SAGE family, XAGE family, NY-ESO-1/LAGE-1, PAME, SSX-2, Melan-A/MART-1, GP100/pme117, TRP-1/-2, P. polypeptide, MC1R, Prostate-specific antigen, β-catenin, BRCA1/2, CML66, Fibronectin, MART-2, TGF-βRII, or VEGF receptors (*e.g*., VEGFR2). The tumor cell antigen to be targeted may be selected for targeting even if it is expressed on normal cells, in some cases. In some embodiments, the tumor cell binding domain targets more than one antigen of a tumor cell.

In particular embodiments, the molecular adapter comprises an antigen receptor target domain. Such construct allows in certain embodiments for the re-direction of an immune effector cell towards a different tumor target antigen (Fig 13 C). The antigen receptor target domain of the molecular adaptor may comprise one or more tumor antigens or functional fragments thereof. Examples of tumor cell antigens include at least 5T4, 8H9, αᵥβ₆ integrin, BCMA, B7-H3, B7-H6, CAIX, CA9, CD19, CD20, CD22, CD30, CD33, CD38, CD44, CD44v6, CD44v7/8, CD70, CD123, CD138, CD171, CEA, CSPG4, EGFR, EGFR family including ErbB2 (HER2), EGFRvIII, EGP2, EGP40, ERBB3, ERBB4, ErbB3/4, EPCAM, EphA2, EpCAM, folate receptor-a, FAP, FBP, fetal AchR, FRα, GD2, G250/CAIX, GD3, Glypican-3 (GPC3), Her2, IL-11Rα, IL-13Rα2, Lambda, Lewis-Y, Kappa, KDR, MAGE, MCSP, Mesothelin, Muc1, Muc16, NCAM, NKG2D Ligands, NY-ESO-1, PRAME, PSC1, PSCA, PSMA, ROR1, SP17, Survivin, TAG72, TEMs, carcinoembryonic antigen, HMW-MAA, AFP, CA-125, ETA, Tyrosinase, MAGE, laminin receptor, HPV E6, E7, BING-4, Calcium-activated chloride channel 2, Cyclin-Bl, 9D7, EphA3, Telomerase, SAP-1, BAGE family, CAGE family, GAGE family, MAGE family, SAGE family, XAGE family, NY-ESO-1/LAGE-1, PAME, SSX-2, Melan-A/MART-1, GP100/pme117, TRP-1/-2, P. polypeptide, MC1R, Prostate-specific antigen, β-catenin, BRCA1/2, CML66, Fibronectin, MART-2, TGF-βRII, or VEGF receptors (*e.g*., VEGFR2) In certain embodiments, the antigen receptor target domain comprises a non-natural target such as *e.g*., proteins or folate conjugated to fluorescein isothiocyanate (folate-FITC; see *e.g.* M.S. Kim et al., J Am Chem Soc. 2015 Mar 4;137(8):2832-5) or the introduction of neo-epitopes into a protein, such as an antibody (see also WO2016168766 and WO/2016/168769A1) and the antigen receptor of the immune cell is directed to the non-natural target. Thereby, the immune effector cell binds to the non-natural target and no endogenous tissue or antigen and is therefore strictly dependent on the presence of the molecular adaptor for activation, hence reducing off-target effects.

The molecular adaptor comprises a cytokine domain, and in particular embodiments the cytokine domain comprises (i) a cytokine or a functional fragment thereof; or (ii) a cytokine receptor or a functional fragment thereof; or (iii) a combination of both (i) and (ii). In particular aspects the cytokine is a pro-survival cytokine. Examples of particular cytokines include cytokines selected from the group consisting of IL-7, IL-15, IL-21, IL-12, IL-18, IL-27, and a mixture thereof. In specific embodiments, the cytokine is IL-15 and/or the cytokine receptor is IL-15R or the alpha subunit thereof (IL15Ra). In specific embodiments, the sushi domain of IL-15Ra is used. In at least some cases, the cytokine is not IL-2, IL-4, IL-6, IL-10, and/or IL-13. In some embodiments, the cytokine domain comprises IE-2Pβ/γ. Thus, in specific embodiments thereof, the cytokine domain comprises IL-15, IL-15Ra and IL-2Rβ/γ. In some cases, the cytokine domain further comprises apolopiprotein.

In some embodiments, the immune effector cells comprise one or more engineered antigen receptors against one or more tumor target antigens. In specific embodiments, the antigen receptor against one or more tumor target antigens is not part of the molecular adaptor but is expressed as a separate molecule by the cell. The antigen receptors may be any kind of engineered receptor, such as chimeric antigen receptor (CAR), chimeric cytokine receptor, alpha beta receptor, gamma delta receptor , a T cell receptor (TCR)and so forth. In preferred embodiments, the antigen receptor is a TCR or a CAR. Examples of tumor target antigens to which the antigen receptors may be directed include at least 5T4, 8H9, αᵥβ₆ integrin, BCMA, B7-H3, B7-H6, CAIX, CA9, CD19, CD20, CD22, CD30, CD33, CD38, CD44, CD44v6, CD44v7/8, CD70, CD123, CD138, CD171, CEA, CSPG4, EGFR, EGFR family including ErbB2 (HER2), EGFRvIII, EGP2, EGP40, ErbB2, ERBB3, ERBB4, ErbB3/4, EPCAM, folate receptor-a, EphA2, EpCAM, FAP, FBP, fetal AchR, FRα, GD2, O-acetyl-GD2, G250/CAIX, GD3, Glypican-3 (GPC3), Her2, HLA-A1+MAGE1, HLA-A1+NY-ESO-1, IL-11Rα, IL-13Rα2, Lambda, Lewis-Y, Kappa, KDR, MAGE, MCSP, Mesothelin, Muc1, Muc16, NCAM, NKG2D Ligands, NY-ESO-1, PRAME, PSC1, PSCA, PSMA, ROR1, SP17, Survivin, TAG72, TEMs, carcinoembryonic antigen, HMW-MAA, AFP, CA-125, ETA, Tyrosinase, laminin receptor, HPV E6, E7, BING-4, Calcium-activated chloride channel 2, Cyclin-Bl, 9D7, EphA3, Telomerase, SAP-1, BAGE family, CAGE family, GAGE family, MAGE family, SAGE family, XAGE family, NY-ESO-1/LAGE-1, PAME, SSX-2, Melan-A/MART-1, GP100/pme117, TRP-1/-2, P. polypeptide, MC1R, Prostate-specific antigen, β-catenin, BRCA1/2, CML66, Fibronectin, MART-2, TGF-βRII, or VEGF receptors (*e.g.*, VEGFR or VEGFR2.

In particular embodiments, the molecular adaptor comprises a tumor cell binding domain targeting a first antigen on the tumor cell and an antigen receptor target domain comprising a second tumor antigen or functional fragment thereof.

In specific embodiments, the antigen of the antigen receptor target domain may be the same as the antigen to which the antigen receptor against one or more tumor target antigens is directed. In cases wherein the antigen of the antigen receptor target domain is a different antigen than the antigen to which the antigen receptor is directed, one of the antigens may be referred to as a first antigen and the other antigen may be referred to as a second antigen.

In particular embodiments, the immune effector cells express one or more antigen receptors against one or more tumor target antigens. In specific embodiments the receptor is non-native to the cell. The antigen receptor may be of any kind, including at least a T cell receptor (TCR) or a chimeric antigen receptor (CAR). When the receptor is a CAR, the CAR may be first generation, second generation, or third or more generation. Accordingly, the CAR may or may not comprise one or more costimulatory domains. A single CAR may target one, two, three, or more tumor target antigens. In particular cases, the engineered CAR has one, two, three, four, or more components, and in some embodiments the one or more components facilitate targeting or binding of the cell to the cancer antigen-comprising cancer cell. In specific embodiments, the CAR comprises an antibody for the cancer antigen, part or all of a cytoplasmic signaling domain, and/or part or all of one or more co-stimulatory molecules, for example endodomains of co-stimulatory molecules. In specific embodiments, the antibody is a single-chain variable fragment (scFv). In certain aspects the antibody is directed at cancer antigens on the cell surface of cancer cells that express an antigen of interest, for example. In certain embodiments, a cytoplasmic signaling domain, such as those derived from the T cell receptor ζ-chain, is employed as at least part of the chimeric receptor in order to produce stimulatory signals for immune effector cell proliferation and effector function following engagement of the chimeric receptor with the target antigen. Examples would include, but are not limited to, endodomains from co-stimulatory molecules such as CD27, CD28, 4-1BB, ICOS, and/or OX40 or the signaling components of cytokine receptors such as IL7 and IL15. In particular embodiments, co-stimulatory molecules are employed to enhance the activation, proliferation, and cytotoxicity of the NKT cells produced by the CAR after antigen engagement. In specific embodiments, the co-stimulatory molecules are CD28, OX40, and 4-1BB. In some embodiments, CD3-zeta is used in combination with CD28, 4-1BB, and/or OX40. In general, an ectodomain of the CAR encompasses a signal peptide, antigen recognition domain, and a spacer that links the antigen recognition domain to the transmembrane domain. The antigen recognition domain may comprise a scFv specific for a particular cancer antigen. However, in cases wherein there are two or more CARs in the same cell, the second CAR may comprise an scFv specific for another particular antigens. Examples of hinge regions for the ectodomain include the CH2CH3 region of immunoglobulin, the hinge region from IgG1, and portions of CD8. The transmembrane region may be of any kind, although in some cases it is CD4 or CD28. In general, the endodomain of the CAR of the disclosure is utilized for signal transmission in the cell after antigen recognition and cluster of the receptors. The most commonly used endodomain component is CD3-zeta. CD3-zeta contains 3 ITAMs and that transmits an activation signal to the T cell after the antigen is bound.

Turning to FIG. 12, a specific interaction between a tumor cell expressing a tumor antigen ((1), that refers to GD2 in this example) and an immune effector cells is depicted. In a specific embodiment, an exemplary molecular adaptor comprises a fusion of CD19 extracellular domain (element (3) or FIG. 12) and a scFv derived from a mAb specific for GD2 (element (2) of FIG. 12), a-GD2CD19). Immune effector cells transduced with a construct containing CAR19 and an a-GD2CD19 adaptor express CAR19 (element (4)) on the cell surface and secrete the molecular adaptor. In this particular example, the antigen component of the molecular adaptor is fused with an activating cytokine domain (*e.g*., IL-15/IL-15Ra, element (5)). This forms an antigen-cytokine complex on the tumor cell surface that is simultaneously recognized by the CAR and the respective cytokine receptor expressed by an immune effector cell. In this particular example, element (6) of FIG. 12 depicts IL-2Rβ/γ.

Referring to FIG. 13, in particular embodiments the immune effector cell and its interaction with a tumor cell may have one of a variety of configurations. In FIG. 13A, the immune effector cell produces a soluble fusion protein referred to as molecular adaptor that targets a first tumor antigen and that also has an antigen receptor targeting a first tumor antigen. The molecular adaptor includes a tumor cell binding domain fused to a cytokine domain (comprising a cytokine and/or a cytokine receptor). This configuration ensures that an immune effector cell receives activation and survival signals on the same target cells for maximal anti-tumor efficacy. In FIG. 13B, the immune effector cell produces a soluble fusion protein referred to as molecular adaptor that targets a second tumor antigen and the cell comprises an antigen receptor that targets a first tumor antigen. The molecular adaptor includes a tumor cell binding domain fused to a cytokine domain (cytokine and/or cytokine receptor). In this configuration, an immune effector cell still receives activation and survival signals from the tumor cells co-expressing both tumor antigens. This configuration may offer increased specificity, since only cells that express both a first and a second antigen would fully activate the immune effector cell. In FIG. 13C, the immune effector cell produces a molecular adaptor targeting a first tumor antigen and the cell also has an antigen receptor. The antigen receptor target domain of the molecular adaptor is targeted by the antigen receptor expressed on the surface of the cell. The molecular adaptor includes a tumor cell binding domain that targets a first antigen of the tumor cell, an antigen receptor target domain, and a cytokine domain. This configuration allows the use of one antigen receptor expressed in immune effector cells to target multiple types of cancer while still receiving both activation and survival signals from the target cell surface.

With respect to the molecular adaptor, the components thereof may or may not be in a particular configuration. For example, in an N-terminal to C-terminal direction, one of the components of the molecular adaptor may be positioned N-terminal or C-terminal to another. In specific embodiments for the molecular adaptor, the tumor cell binding domain and the antigen receptor target domain may be linked by a hinge region and/or (GGGGS (SEQ ID NO:15))₁ (GGGGS)₂ (GGGGS)₃ or (GGGGS)₄ linker. In specific embodiments, the hinge region is from CD8, IgG1, IgG2, IgG3, or IgG4, for example. In certain embodiments, the hinge region is from IgG4.

Engineered immune effector cells that produce certain non-natural proteins are encompassed by the disclosure. In specific cases, immune effector cells that produce (1) an antibody or its functional fragment that targets a first tumor antigen or a second tumor antigen, wherein the antibody is linked to (a) a cytokine, (b) a cytokine receptor, or (c) a cytokine linked to its receptor. In other cases, immune effector cells produce an antibody or its functional fragment that targets a first tumor antigen, wherein the antibody is linked to a second tumor antigen, which in turn is linked to (a) a cytokine, (b) a cytokine receptor, or (c) a cytokine linked to its receptor. In any case for immune effector cells encompassed herein, the cell may comprise a CAR or engineered TCR (or natural TCR) that targets a first tumor antigen or a second tumor antigen.

In particular embodiments, the immune effector cells harbor one or more non-native (that may be recombinant) nucleic acids that are engineered to encode one or more gene products. In particular embodiments the cells comprise one or more non-natural (that may be recombinant) expression vectors, and the vector(s) may comprise one or more expression constructs that encode one or more particular gene products. The expression vector may be a viral vector or a non-viral vector. In cases wherein the vector is a viral vector, the viral vector may be a retroviral, lentiviral, adenoviral, or adeno-associated viral vector. In cases wherein the vector is a non-viral vector, the vector may be a plasmid, an isolated DNA or RNA, a transposon, or other mobile genetic element. In particular aspects one vector in the immune effector cell encodes a molecular adaptor and an antigen receptor, although in other cases the molecular adaptor and the antigen receptor are expressed from different vectors. When the molecular adaptor and the antigen receptor are expressed from different vectors, the vectors may or may not be of the same type.

In one embodiment there is a molecular adaptor for redirecting cells expressing antigen receptors, comprising (a) a tumor cell binding domain, (b) a cytokine domain comprising (i) a cytokine or a functional fragment thereof; or (ii) a cytokine receptor or a functional fragment thereof; or (iii) a combination of both (i) and (ii), and (c) an antigen receptor target domain. In specific configurations, the cytokine domain in the molecular adaptor is positioned between the tumor cell binding domain and the antigen receptor domain. As described above, the tumor cell binding domain may, *e.g*., comprise an antibody, an antibody fragment or a non-antibody scaffold, and in preferred embodiments an antibody fragment being a scFv. In specific cases, the tumor cell binding domain targets an antigen expressed on the surface of a tumor cell of any kind. As described above, the cytokine domain may comprise any cytokine, including pro-survival cytokines such as IL-7, IL-15 or IL-21, for example. In a specific example for the molecular adaptor, the cytokine is IL-15 and/or the cytokine receptor is IL-15R or the alpha subunit thereof (IL15Ra). The antigen receptor target domain of the soluble protein may comprise a tumor antigen or a functional fragment thereof, such as CD19, for example. In a specific embodiment, the molecular adaptor comprises a tumor cell binding domain targeting a first antigen on the tumor cell and an antigen receptor target domain comprising a second tumor antigen or functional fragment thereof. The tumor cell binding domain and the antigen receptor target domain of the molecular adaptor may be linked by a hinge region, such as one from IgG1, IgG2, IgG3, or IgG4, preferably from IgG4.

In one embodiment there is an antigen receptor directed against a molecular adaptor encompassed by the disclosure. In particular cases the antigen receptor is a TCR (for example, for molecules related to MHC antigens on cell surfaces) or a CAR, for example, and comprises a moiety that binds a component of the molecular adaptor. In specific cases said antigen receptor binds an antigen receptor target domain of the molecular adaptor, such as a tumor antigen or a functional fragment thereof. In alternative cases the antigen receptor binds a component of the molecular adaptor that is not the antigen receptor target domain.

Embodiments of the disclosure include expression vectors that encode a molecular adaptor encompassed by the disclosure, including a molecular adaptor that comprises (a) a tumor cell binding domain; (b) a cytokine domain; and (c) an antigen receptor target domain. The expression vectors may be viral or non-viral. In cases wherein the expression vectors encoding the soluble protein are viral, they may be retroviral, lentiviral, adenoviral, or adeno-associated. In cases wherein the expression vector is non-viral, the expression vector may be a plasmid, an isolated DNA or RNA, a transposon, or other mobile genetic element. In a particular embodiment, there is an expression vector encoding a molecular adaptor comprising (i) a tumor cell binding domain, and (ii) a cytokine domain; and the expression vector encodes (b) an antigen receptor.

In some cases the expression vector encodes a molecular adaptor and also encodes an antigen receptor on the same vector. In such cases, the expression of the molecular adaptor and the expression of the antigen receptor may or may not be directed by the same regulatory region (such as promoter). In cases wherein the expression of the molecular adaptor and the antigen receptor is regulated by different regulatory regions, their expression may be controlled by an internal ribosome entry site (IRES) or a 2A sequence. In particular cases, an IRES element or a 2A sequence is located between the molecular adaptor and the antigen receptor. In at least some cases, both a CAR (and/or engineered TCR) and a molecular adaptor molecule can be effectively expressed in immune effector cells using a bi- or tri-cistronic vector, such as a retroviral vector.

In particular embodiments, there is a cell comprising the expression vector, or a plurality of cells comprising the expression vector. As described above, the cells may be of any kind, but in particular embodiments the cells are immune effector cells, preferably T cells, NKT cells, or NK cells, or a mixture thereof. The cells in reference to a particular individual may be autologous or allogeneic.

Embodiments of the disclosure include one or more pharmaceutical compositions comprising one or more compositions of the disclosure. In particular embodiments, the pharmaceutical composition comprises an immune effector cell (or plurality thereof) encompassed by the disclosure. In particular embodiments the pharmaceutical composition comprises a molecular adaptor encompassed by the disclosure. In some embodiments a pharmaceutical composition comprises immune effector cells and molecular adaptors. In at least one case there is a pharmaceutical composition comprising an immune effector cell that comprises an expression vector encoding a molecular adaptor and/or an antigen receptor against a target antigen. In cases where a pharmaceutical composition comprises a plurality of immune effector cells as described herein, the pharmaceutical composition may comprise a certain amount of the immune effector cells, such as 1x10⁷/m² to 1x10⁹/m². In cases wherein the pharmaceutical composition comprises molecular adaptor, the composition may be formulated to comprise a certain amount of molecular adaptor, such as 10pg/mL to 50mg/mL, for example.

In one embodiment, there is an immune effector cell, wherein the cell produces (1) an antibody (such as a scFv) or functional fragment thereof that targets a first tumor antigen or a second tumor antigen, wherein the antibody is linked to (a) a cytokine, or (b) a cytokine receptor, or (c) a cytokine linked to its receptor; or (2) an antibody or functional fragment thereof that targets a first tumor antigen, wherein the antibody or functional fragment thereof is linked to a second tumor antigen as an antibody-second tumor antigen complex, wherein the complex is linked to (a) a cytokine or (b) a cytokine receptor or (c) a cytokine linked to its receptor. In at least some cases, the cell comprises a TCR and/or CAR that targets a first tumor antigen or a second tumor antigen. In specific embodiments, (1) or (2) are produced from an expression vector therein. The expression vector is a viral vector (including at least retroviral, lentiviral, adenoviral, or adeno-associated viral vector) or a non-viral vector, in some cases. In cases where the immune effector cell and expresses a CAR, the CAR may comprise one, two, three, or more co-stimulatory domains. In some cases for the embodiment in (2), all or a functional part of the second tumor antigen comprises an extracellular domain of the second tumor antigen. In some cases for the embodiment in (2), the antibody or functional fragment thereof that targets a first tumor antigen and the second tumor antigen are linked by a hinge region (such as from CD8α, IgG1, IgG2, IgG3, or IgG4). In some cases of the particular immune effector cell embodiment, the cytokine is a pro-survival cytokine, such as IL-15, IL-7 or IL-21.

In one embodiment, there is a system, comprising one or more polynucleotides comprising: (1) a first sequence that encodes a CAR that targets a first tumor antigen; and (2) a second sequence that encodes a pro-survival cytokine (IL-15, IL-7 or IL-21, for example) or a fusion between a pro-survival cytokine and a receptor for the pro-survival cytokine, and one of the following: (a) an antibody or functional fragment thereof (such as an scFv) that targets the first or a second tumor antigen; or (b) all or a functional part of the first tumor antigen fused to the antibody or functional fragment that targets the second tumor antigen. In the molecular adaptor of embodiment (2), element (b) may be proximal to the fusion of element (c). In the molecular adaptor of embodiment (2), element (b) is adjacent to the fusion element (c). In particular embodiments, the polynucleotide is further defined as an expression vector, including a viral vector (retroviral, lentiviral, adenoviral, or adeno-associated viral vector) or a non-viral vector. The CAR may comprise one, two, or three, or more co-stimulatory domains. In some cases, all or a functional part of the first tumor antigen comprises an extracellular domain of the first tumor antigen. In some cases, the (a) an antibody or functional fragment thereof that targets a second tumor antigen and the (b) all or a functional part of the first tumor antigen are linked by a hinge region (such as from CD8, IgG1, IgG2, IgG3, or IgG4). In some cases, the polynucleotide is expressed in an immune effector cell, such as an NK cell, an NKT cell, or a T cell.

As an illustrative embodiment, NKT cells are transduced with a construct that expresses CAR19 on the cell surface and secretes an a-GD2.CD19 adaptor. The secreted molecular adaptor is sufficient to convert CD19-negative GD2-positive but not GD2-negative tumor cells into CD19-positive tumor cells. The CAR19 binds to the a-GD2.CD19 adaptor which in turn binds to GD2 expressed on the tumor cell. In doing so, NKT cells expressing CAR19 and an a-GD2.CD19 adaptor effectively and specifically kill GD2-positive tumor cells *in vitro* or *in vivo.* In some cases, neither IL-15 nor IL-15R are required for *in vitro* killing of GD2-positive tumor cells by NKT cells expressing CAR19 and an a-GD2.CD19 adaptor, although in other cases IL-15 and/or IL-15R are utilized. In particular embodiments, though, NKT cells expressing CAR19 and the a-GD2.CD19 adaptor with fused IL-15 and IL-15R have potent therapeutic activity for GD2-positive cancers, including GD2-positive neuroblastoma in mice as an example. In at least some cases, molecular adaptors without IL-15 and/or IL-15R are ineffective *in vivo.*

In other illustrative embodiments, IL-15 and IL-15R are connected *via* a linker in a defined orientation and fused to the target antigen (*e.g*., CD19) in the molecular adaptor construct so that CAR19 NKT cells will recognize CD19 *via* CAR and IL-15/IL-15R complex *via* the common IL-2/IL-15Rβγ receptor in close molecular proximity on the tumor cell surface.

In some embodiments, the molecular adaptor comprises a tumor cell binding domain targeting CD 19 and a IL-15 as cytokine domain. In preferred embodiments thereof, the tumor cell binding domain is a scFv targeting CD19. In some embodiments, such molecular adaptor further comprises an antigen receptor target domain as described herein.

In some embodiments, the molecular adaptor comprises a tumor cell binding domain targeting CD19 and a IL-15R, more preferably IL-15Ra as cytokine domain. In preferred embodiments thereof, the tumor cell binding domain is a scFv targeting CD19. In some embodiments, such molecular adaptor further comprises an antigen receptor target domain as described herein.In some embodiments, the molecular adaptor comprises a tumor cell binding domain targeting CD19 and IL-15 linked to IL-15R, more preferably IL-15Ra as cytokine domain. In preferred embodiments thereof, the tumor cell binding domain is a scFv targeting CD19. In some embodiments, such molecular adaptor further comprises an antigen receptor target domain as described herein.

In some embodiments, the molecular adaptor comprises a tumor cell binding domain targeting CD24 and a IL-15 as cytokine domain. In preferred embodiments thereof, the tumor cell binding domain is a scFv targeting CD24. In some embodiments, such molecular adaptor further comprises an antigen receptor target domain as described herein.

In some embodiments, the molecular adaptor comprises a tumor cell binding domain targeting CD24 and a IL-15R, more preferably IL-15Ra as cytokine domain. In preferred embodiments thereof, the tumor cell binding domain is a scFv targeting CD24. In some embodiments, such molecular adaptor further comprises an antigen receptor target domain as described herein.

In some embodiments, the molecular adaptor comprises a tumor cell binding domain targeting CD24 and IL-15 linked to IL-15R, more preferably IL-15Ra, as cytokine domain. In preferred embodiments thereof, the tumor cell binding domain is a scFv targeting CD24. In some embodiments, such molecular adaptor further comprises an antigen receptor target domain as described herein.

In some embodiments, the molecular adaptor comprises a tumor cell binding domain targeting CD56 and a IL-15 as cytokine domain. In preferred embodiments thereof, the tumor cell binding domain is a scFv targeting CD56. In some embodiments, such molecular adaptor further comprises an antigen receptor target domain as described herein.

In some embodiments, the molecular adaptor comprises a tumor cell binding domain targeting CD56 and a IL-15R, more preferably IL-15Ra as cytokine domain. In preferred embodiments thereof, the tumor cell binding domain is a scFv targeting CD56. In some embodiments, such molecular adaptor further comprises an antigen receptor target domain as described herein.

In some embodiments, the molecular adaptor comprises a tumor cell binding domain targeting CD56 and IL-15 linked to IL-15R, more preferably IL-15Ra, as cytokine domain. In preferred embodiments thereof, the tumor cell binding domain is a scFv targeting CD56. In some embodiments, such molecular adaptor further comprises an antigen receptor target domain as described herein.

In some embodiments, the molecular adaptor comprises a tumor cell binding domain targeting CD22 and a IL-15 as cytokine domain. In preferred embodiments thereof, the tumor cell binding domain is a scFv targeting CD22. In some embodiments, such molecular adaptor further comprises an antigen receptor target domain as described herein.

In some embodiments, the molecular adaptor comprises a tumor cell binding domain targeting CD22 and a IL-15R, more preferably IL-15Ra as cytokine domain. In preferred embodiments thereof, the tumor cell binding domain is a scFv targeting CD22. In some embodiments, such molecular adaptor further comprises an antigen receptor target domain as described herein.

In some embodiments, the molecular adaptor comprises a tumor cell binding domain targeting CD22 and IL-15 linked to IL-15R, more preferably IL-15Ra, as cytokine domain. In preferred embodiments thereof, the tumor cell binding domain is a scFv targeting CD22. In some embodiments, such molecular adaptor further comprises an antigen receptor target domain as described herein.

In some embodiments, the molecular adaptor comprises a tumor cell binding domain targeting BCMA and a IL-15 as cytokine domain. In preferred embodiments thereof, the tumor cell binding domain is a scFv targeting BCMA. In some embodiments, such molecular adaptor further comprises an antigen receptor target domain as described herein.

In some embodiments, the molecular adaptor comprises a tumor cell binding domain targeting BCMA and a IL-15R, more preferably IL-15Ra, as cytokine domain. In preferred embodiments thereof, the tumor cell binding domain is a scFv targeting BCMA. In some embodiments, such molecular adaptor further comprises an antigen receptor target domain as described herein.

In some embodiments, the molecular adaptor comprises a tumor cell binding domain targeting BCMA and IL-15 linked to IL-15R, more preferably IL-15Ra, as cytokine domain. In preferred embodiments thereof, the tumor cell binding domain is a scFv targeting BCMA. In some embodiments, such molecular adaptor further comprises an antigen receptor target domain as described herein.

In some embodiments, the molecular adaptor comprises a tumor cell binding domain targeting GD2 and a IL-15 as cytokine domain. In preferred embodiments thereof, the tumor cell binding domain is a scFv targeting GD2. In some embodiments, such molecular adaptor further comprises an antigen receptor target domain as described herein.

In some embodiments, the molecular adaptor comprises a tumor cell binding domain targeting GD2 and a IL-15R, more preferably IL-15Ra, as cytokine domain. In preferred embodiments thereof, the tumor cell binding domain is a scFv targeting GD2. In some embodiments, such molecular adaptor further comprises an antigen receptor target domain as described herein.

In some embodiments, the molecular adaptor comprises a tumor cell binding domain targeting GD2 and IL-15 linked to IL-15R, more preferably IL-15Ra, as cytokine domain. In preferred embodiments thereof, the tumor cell binding domain is a scFv targeting GD2. In some embodiments, such molecular adaptor further comprises an antigen receptor target domain as described herein.

### GENERAL EMBODIMENTS

It is demonstrated herein that immune effector cells (NKT cells, as examples) engineered to express an effective CAR against one tumor antigen, CD19 can be effectively redirected against tumor cells expressing another antigen, GD2, using the molecular adaptor approach. The molecular adaptor comprises a fusion of CD19 extracellular domain and a scFv derived from a mAb specific for GD2 (aGD2CD19). NKT cells transduced with a construct containing CAR19 and an aGD2CD19 adaptor express CAR19 on the cell surface and secrete the molecular adaptor. The secreted molecular adaptor is sufficient to convert CD19-negative GD2-positive but not GD2-negative neuroblastoma cells into CD19-positive cells and enable *in vitro* cytotoxicity of CAR19 NKT cells against GD2-positive tumor cells. Even more potent anti-tumor activity *in vivo* has been achieved *via* fusing the antigen part of the molecular adaptor (CD19) with IL-15 and IL-15Ra, connected *via* a linker in a defined orientation, forming an antigen-cytokine complex. Moreover, secretion of IL-15 or IL15-IL15Ra complex by CAR19 NKT cells separately from aGD2.CD19 adaptor did not significantly enhance *in vivo* anti-tumor activity compared to the molecular adaptor alone. Therefore, in order for the molecular adaptor to be effective *in vivo,* IL15, IL15Ra, or IL15-IL15Ra complex or a similar pro-survival cytokine (molecule) in at least some embodiments must be linked to an antigen binding domain, so that it can be co-presented with a target antigen on the tumor cell surface. This allows CAR-expressing effector cells to recognize a target antigen and a cytokine in the close molecular proximity on the tumor cell surface. This embodiment of engineering immune effector cells to express a tumor-specific CAR and to deliver a target tumor antigen with a fused cytokine is novel and effective in an *in vivo* model of neuroblastoma, and it is broadly applicable to immunotherapy of solid tumors and other cancers and diseases, including non-cancerous diseases.

These results establish that an immune effector cell such as NKT cell, engineered to express an antigen receptor, such as CAR against one antigen such as CD19, can be effectively redirected against another antigen, e.g. a tumor antigen such as GD2 using the molecular adaptor approach described herein.

The evidence indicates that co-presentation of a target antigen for CAR recognition (provides signal-1 and -2) and a pro-survival cytokine (signal-3) for the effector cell, where presentation can occur from the same tumor cell surface is useful for enabling effective CAR-mediated immunotherapy of solid tumors. While the inventors achieved such presentation on the tumor cell surface using a specific a-GD2.CD19 adaptor as a delivery vehicle and a IL-15/L-15R as a cytokine, it was considered that similarly or even more effective results can be achieved in the following ways:
1) a-GD2.CD19 adaptor as a delivery vehicle can be replaced with a scFv directly targeting an antigen highly expressed on tumor cells, even if it is also expressed in some normal tissues (recognition of a cytokine without a CAR/TCR target is not expected to trigger cytotoxicity). In the case of neuroblastoma, such antigens include CD56, CD24, CD171, and/or H7-B3, for example. Such scFv would be fused to IL-15, IL-15Ra, or IL-15/L-15Ra (as examples), achieving a similar molecular adaptor that would be recognized by CAR (*e.g.* CARGD2)-redirected immune cells on the same tumor cells. Such constructs for expression in NKT cells may be utilized in neuroblastoma models, for example.
2) IL-15 or IL-15Ra may be replaced with other pro-survival cytokines or cytokine receptors such as IL-7, IL-12, IL-18, IL-27, IL-33, or IL-21, as well as their respective receptors, for example.
3) In some embodiments, instead of engineering molecular adaptors to be expressed and secreted by antigen receptor-expressing immune effector cells, such molecular adaptors could be pre-made as fusion proteins for systemic delivery in combination with any CAR/TCR cell therapy. For example, mAbs targeting the same antigens as described in item 1 above could be fused with IL-15, IL-15Ra, or IL15-IL15-Ra (or another cytokine as in item 2) and used as an off-the-shelf therapy in combination with any adoptive immunotherapy of cancers, including solid tumors.

### II. Methods of Producing the Immune Effector Cells and Adapting a Cancer Therapy

Immune effector cells encompassed herein are non-natural at least because they are engineered to express an engineered antigen receptor (such as TCR or a CAR) that targets a particular antigen and/or because they are engineered to produce a molecular adaptor as described above. The immune effector cells may be engineered by standard recombinant technology in at least some cases.

In particular embodiments, the immune effector cells may be genetically modified prior to delivery to an individual in need thereof. The immune effector cells may be modified after TCR-stimulation and co-stimulation; in particular embodiments the genetic modification occurs within 1, 2, 3, 4, 5, 7, 8, 9, 10, 11, 12, or more days after stimulation (and this may depend on the type of transduction and type of effector cells used; for example with retroviral vectors it may be within 2 days for T cells and 7-9 days for NKT cells).

The genetic modification of the immune effector cells can occur by the hand of man, and in particular embodiments the genetic modification renders the cells able to specifically target one or more cancer cells, such as cancer cells that express a particular antigen, for example. In specific embodiments, the modification provides the immune effector cells with a particular non-natural receptor for a certain cancer antigen.

In particular cases, immune effector cells include a CAR or TCR that is chimeric and/or non-natural, engineered at least in part by the hand of man, and directed to a particular cancer antigen of interest.

In any therapeutic methods of the disclosure, cells of the cancer may lack expression of a particular tumor antigen, such as lack expression of an antigen to which a certain antigen receptor on an immune effector cell may be directed. In such cases, a molecular adaptor bridges the tumor cell with the immune effector cell because the molecule adaptor comprises a component that can bind the tumor cell and a component that can bind the immune effector cell. Therefore, the molecular adaptor may allow certain immune effector cells to be used universally so long as the molecular adaptor corresponds to the certain immune effector cells in having a component that binds the certain immune effector cells. In one example, immune effector cells expressing an antigen receptor that binds a first tumor antigen may be produced and used universally with any type of cancer because they are used with a variety of interchangeable molecular adaptors having at least one component that binds the antigen receptor of the immune effector cells; in such cases, the molecular adaptors have a second component that binds a second antigen of a cancer of interest.

The disclosure encompasses methods for adapting one type of adoptive immunotherapy to be universally applied to any and all types of cancer, regardless of the type of antigens expressed on the intended recipient cancer cells. Such methods utilize existing, prepared immune effector cells that express an artificial antigen receptor as described above that targets a particular antigen and modifies those cells to produce a molecular adaptor that allows use of the immune effector cells for cancers that express antigens other than that particular antigen. In effect, a molecular adaptor produced by the immune effector cells and having bispecific targeting moieties is employed to bridge cells engineered to target the first antigen with tumor cells expressing a second, non-identical antigen. In alternative cases, prepared immune effector cells that express an artificial antigen receptor (CAR or TCR, for example) that targets a first antigen are used in conjunction with proteins that are exogenously provided (that is, not produced by the immune effector cells themselves) and that have bispecific targeting moieties that bridge the immune effector cells engineered to target the first antigen with tumor cells expressing a second, non-identical antigen.

Thus, embodiments of the disclosure include methods of adapting a cancer therapy to a cancer expressing a first tumor antigen, comprising the steps of (a) providing or obtaining an immune effector cell or plurality thereof comprising a non-natural antigen receptor that targets a second tumor antigen; and (b) providing or obtaining a molecular adaptor comprising a tumor cell binding domain that binds the first tumor antigen and an antigen receptor target domain that binds the antigen receptor that targets a second tumor antigen, optionally wherein the molecular adaptor comprises (i) a cytokine or a functional fragment thereof; or (ii) a cytokine receptor or a functional fragment thereof; or (iii) a combination of both (i) and (ii), and wherein the providing or obtaining of the molecular adaptor in step (b) is determined by the presence of expression of the first antigen in the cancer cells of the cancer being treated.

In some embodiments, the methods of the disclosure include a step of administering a purified molecular adaptor as described above to an individual.

Embodiments of the disclosure include methods of adapting a cancer therapy to a cancer expressing a first tumor antigen, comprising the steps of (a) providing or obtaining an immune effector cell or plurality thereof comprising a non-natural antigen receptor that targets a second tumor antigen; and (b) transfecting said cells with a polynucleotide that encodes a molecular adaptor comprising: (1) a tumor cell binding domain that targets the first antigen, and (2) a cytokine domain comprising: (i) a cytokine or a functional fragment thereof; or (ii) a cytokine receptor or a functional fragment thereof; or (iii) a combination of both (i) and (ii).

### III. Methods of Using the Immune Effector Cells

By way of illustration, individuals with cancer or individuals susceptible to cancer or suspected of having cancer may be treated as described herein, as well as individuals with a disease other than cancer or susceptible to or suspected of having a disease other than cancer. Immune effector cells modified as described herein may be administered to the individual and retained for extended periods of time. The individual may receive one or more administrations of the cells. In some embodiments, the genetically modified cells are encapsulated to inhibit immune recognition and placed at the site of the tumor(s) or affected area(s).

In various embodiments the expression constructs, nucleic acid sequences, vectors, host cells and/or pharmaceutical compositions comprising the same are used for the prevention, treatment or amelioration of a disease, including a cancerous disease, such as a tumorous disease. In particular embodiments, the pharmaceutical composition of the present disclosure may be particularly useful in preventing, ameliorating and/or treating disease, such as cancer and including cancer having solid tumors, for example.

As used herein "treatment" or "treating," includes any beneficial or desirable effect on the symptoms or pathology of a disease or pathological condition, and may include even minimal reductions in one or more measurable markers of the disease or condition being treated, *e.g*., cancer. Treatment can involve optionally either the reduction or amelioration of symptoms of the disease or condition, or the delaying of the progression of the disease or condition. "Treatment" does not necessarily indicate complete eradication or cure of the disease or condition, or associated symptoms thereof.

As used herein, "prevent," and similar words such as "prevented," "preventing" *etc.,* indicate an approach for preventing, inhibiting, or reducing the likelihood of the occurrence or recurrence of, a disease or condition, *e.g*., cancer. It also refers to delaying the onset or recurrence of a disease or condition or delaying the occurrence or recurrence of the symptoms of a disease or condition. As used herein, "prevention" and similar words also includes reducing the intensity, effect, symptoms and/or burden of a disease or condition prior to onset or recurrence of the disease or condition.

In particular embodiments, the present disclosure contemplates, in part, cells harboring expression constructs, nucleic acid molecules and/or vectors and/or purified molecular adaptors that can administered either alone or in any combination with another therapy, and in at least some aspects, together with a pharmaceutically acceptable carrier or excipient. In certain embodiments, prior to administration of the cells, said nucleic acid molecules, vectors may be stably integrated into the genome of the cells (for example, using CRISPR methods). In specific embodiments, viral vectors may be used that are specific for certain cells or tissues and persist in said cells. Suitable pharmaceutical carriers and excipients are well known in the art. The compositions prepared according to the disclosure can be used for the prevention or treatment or delaying the above identified diseases.

Furthermore, the disclosure relates to a method for the prevention, treatment or amelioration of a disease, such as a cancerous (including tumorous) disease comprising the step of administering to a subject in need thereof an effective amount of engineered cells harboring an antigen receptor and optionally a molecular adaptors is described herein and a chemotherapy resistance molecule, nucleic acid sequence that encodes them, vector(s) that encodes them, as contemplated herein and/or produced by a process as contemplated herein.

Possible indications for administration of the composition(s) of the exemplary modified immune cells are diseases, such as cancerous diseases, including tumorous diseases. Specific examples are but are not limited to neuroblastoma, medulloblastoma, breast cancer, cervical cancer, ovary cancer, endometrial cancer, melanoma, bladder cancer, lung cancer, pancreatic cancer, colon cancer, prostate cancer, hematopoietic tumors of lymphoid lineage, leukemia, acute lymphocytic leukemia, chronic lymphocytic leukemia, B-cell lymphoma, Burkitt's lymphoma, multiple myeloma, Hodgkin's lymphoma, Non-Hodgkin's lymphoma, myeloid leukemia, acute myelogenous leukemia (AML), chronic myelogenous leukemia, thyroid cancer, thyroid follicular cancer, myelodysplastic syndrome (MDS), tumors of mesenchymal origin, fibrosarcoma, rhabdomyosarcomas, melanoma, uveal melanoma, teratocarcinoma, neuroblastoma, glioma, glioblastoma, benign tumor of the skin, renal cancer, anaplastic large-cell lymphoma, esophageal squamous cells carcinoma, hepatocellular carcinoma, follicular dendritic cell carcinoma, cancers of the bile duct, gall bladder cancer, intestinal cancer, muscle-invasive cancer, seminal vesicle tumor, epidermal carcinoma, spleen cancer, bladder cancer, head and neck cancer, cancers of the genito-urinary tract, kidney cancer, gastric cancer, cancer of the small intestine, stomach cancer, liver cancer, bone cancer, brain cancer, cancer of the retina, biliary cancer, small bowel cancer, salivary gland cancer, cancer of uterus, cancer of testicles, cancer of connective tissue, prostatic hypertrophy, myelodysplasia, Waldenstrom's macroglobinaemia, nasopharyngeal, neuroendocrine cancer myelodysplastic syndrome, mesothelioma, angiosarcoma, Kaposi's sarcoma, carcinoid, oesophagogastric, fallopian tube cancer, peritoneal cancer, papillary serous mullerian cancer, malignant ascites, gastrointestinal stromal tumor (GIST), and a hereditary cancer syndrome selected from Li-Fraumeni syndrome and Von Hippel-Lindau syndrome (VHL).

Exemplary indications for administration of the composition(s) of cells are any disease, such as cancerous diseases, including any malignancies that express a particular antigen, for example. In addition, it includes malignancies that aberrantly express other tumor antigens and those may also be targeted. The administration of the composition(s) of the disclosure is useful for all stages and types of cancer, including for minimal residual disease, early cancer, advanced cancer, and/or metastatic cancer and/or refractory cancer, for example.

The disclosure further encompasses co-administration protocols with other compounds, e.g. bispecific antibody constructs, targeted toxins or other compounds, which act *via* immune cells. The clinical regimen for co-administration of the inventive compound(s) may encompass co-administration at the same time, before and/or after the administration of the other component. Particular combination therapies include chemotherapy, radiation, surgery, hormone therapy, or other types of immunotherapy.

Embodiments relate to a kit comprising one or more immune effector cells as described herein, a nucleic acid sequence as described herein, a vector as described herein, a purified molecular adaptor as described herein and/or a host as described herein. It is also contemplated that the kit of this disclosure comprises a pharmaceutical composition as described herein above, either alone or in combination with further medicaments to be administered to an individual in need of medical treatment or intervention.

The immune effector cells that have been modified with the construct(s) may be grown in culture under selective conditions and cells that are selected as having the construct may then be expanded and further analyzed, using, for example the polymerase chain reaction or flow cytometry for determining the presence of the construct(s) in the host cells. Once the modified host cells have been identified, they may then be used as planned, *e.g.*, expanded in culture or introduced into a host organism.

Depending upon the nature of the cells, the cells may be introduced into a host organism, e.g., a mammal, in a wide variety of ways. The cells may be introduced at the site of the tumor, in specific embodiments, although in alternative embodiments the cells home to the cancer or are modified to home to the cancer. The number of cells that are employed will depend upon a number of circumstances, the purpose for the introduction, the lifetime of the cells, the protocol to be used, for example, the number of administrations, the ability of the cells to multiply, the stability of the recombinant construct, and the like. The cells may be applied as a dispersion, generally being injected at or near the site(s) of interest, or they may be injected or infused into the bloodstream, or into the peritoneal cavity (as examples), or at any other suitable site(s). The cells may be in a physiologically-acceptable medium.

The DNA introduction need not result in integration in every case. In some situations, transient maintenance of the DNA introduced may be sufficient. In this way, one could have a short term effect, where cells could be introduced into the host and then turned on after a predetermined time, for example, after the cells have been able to home to a particular site.

The cells may be administered as desired. Depending upon the response desired, the manner of administration, the life of the cells, the number of cells present, various protocols may be employed. The number of administrations will depend upon the factors described above at least in part.

Embodiments of the disclosure encompass methods of treating any kind, stage, and type of cancer in an individual by providing an effective amount of any one or more compositions encompassed by the disclosure to the individual. The cancer may be primary, metastatic, refractory, recurrent, and so forth. In specific embodiments the cancer is of the breast, brain, lung, colon, skin, liver, kidney, stomach, spleen, pancreas, prostate, ovary, endometrium, testes, blood, bone, and so forth. The individual being treated with one or more compositions encompassed herein may or may not have received one or more therapies prior to exposure to the therapy of this disclosure. In particular embodiments, a therapeutically effective amount of immune effector cells are provided to an individual with cancer. The individual may have been diagnosed as having cancer by any suitable type of method, such as cytological, histological, and so forth, and may include analysis of DNA, RNA, and/or protein from one or more samples of the individual. In specific cases, diagnosis may include one or more of assaying for one or more particular compounds in fluid or tissue (for example, in the blood, urine, semen, and/or cerebrospinal fluid), imaging tests, CT scans, PET scans, and/or biopsy, including bone marrow aspiration in specific cases.

In some cases of the disclosure, administration of the immune effector cells is systemic, and the administration may be parenteral in some cases. In particular embodiments, the immune effector cells are administered locally to the tumor.

Embodiments of the disclosure include antigen-negative tumors that are rendered susceptible to antigen-redirected immune effector cells *via* a molecular adaptor comprising a fusion of the extracellular domain of the antigen and a scFv specific for an antigen expressed by the tumor cells.

In one embodiment to counteract suppressive microenvironments of solid tumors, a molecular adaptor could be linked to or co-expressed with IL-15 alone and/or with IL-15Ra (as an example of cytokines also including IL-7, IL-12, IL18, IL-21, IL-27, or IL-33) to support survival and function of the immune effector cells in tumor tissues.

In one embodiment, there are methods of treating cancer in an individual, wherein a therapeutically effective amount of immune effector cells of the disclosure is administered to the individual with the cancer. In such embodiments, the immune effector cells secrete a molecular adaptor as described herein. The immune effector cells may be administered to the individual once or more than once. In cases where the immune effector cells are administered to the individual more than once, the duration of time between administrations may be of any suitable time, including on the order of 1-24 hours, 1-7 days, 1-4 weeks, 1-12 months, or 1 or more years. In some cases, immune effector cells expressing the same molecular adaptor and/or antigen receptor are administered to the individual upon more than one administration. In other cases, immune effector cells expressing a first molecular adaptor and/or first antigen receptor are administered in one administration and immune effector cells expressing a second molecular adaptor and/or second antigen receptor are administered in a subsequent administration of the cells.

In one embodiment, there is a method of treating cancer in an individual, comprising administering a therapeutically effective amount of a molecular adaptor as described hereinthat comprises a tumor cell binding domain, a cytokine domain, and an antigen receptor target domain (such as part or all of an antigen) and also administering a therapeutically effective amount of cells expressing an antigen receptor, such as immune effector cells expressing an antigen receptor of the disclosure. In such cases, the molecular adaptor is provided to the individual without being produced by cells administered to the individual *for in vivo* production of the molecular adaptor. As such, methods of the disclosure encompass adoptive immunotherapy in conjunction with administration of a pharmaceutical composition that comprises the molecular adaptor. In some cases, administration of the molecular adaptor and administration of cells expressing an antigen receptor occurs at the same time, although in other cases the administrations occur at different times. When the administration of the molecular adaptor and cells expressing an antigen receptor occur at the same time, the administrations may or may not be of the same administration route. When the molecular adaptor and cells expressing an antigen receptor occur at different times, the administrations may or may not be of the same administration route. When the administration of the molecular adaptor and cells expressing an antigen receptor occur at different times, the duration of time between their administrations may be on the order of 1-60 seconds, 1-60 minutes, 1-24 hours, 1-7 days, 1-4 weeks, 1-12 months, or 1 or more years in duration. In at least some cases, the antigen to which the antigen receptor is targeted is the same antigen as the antigen receptor target domain. In particular embodiments, the antigen to which the antigen receptor is targeted is a different antigen than an antigen on a tumor cell to be treated.

In some embodiments, there is a method of treating cancer in an individual, comprising administering a therapeutically effective amount of a molecular adaptor comprising (a) a tumor cell binding domain (such as an antibody fragment, including a scFv) targeting a first or a second antigen on a tumor cell and (b) a cytokine domain, and administering a therapeutically effective amount of cells expressing an antigen receptor against a first antigen on the tumor cell. In such cases, methods of the disclosure encompass adoptive immunotherapy in conjunction with administration of a molecular adaptor without being produced *in vivo* by cells provided to the individual.

In cases wherein molecular adaptor (s) are administered to individuals in need thereof without being produced *in vivo* in the individual, the molecular adaptor may be administered by subcutaneous, intraperitoneal or intravenous injection, for example. In cases wherein immune effector cells are provided to an individual, the cells may be administered by intraperitoneal or intravenous injection, for example. The soluble proteins may be delivered once to the individual or multiple times. When multiple administrations of the soluble proteins to the individual occur, subsequent administrations may or may not be the same soluble protein as in earlier administration(s).

In any therapeutic methods of the disclosure, the individual receiving the therapy may be receiving, may have received, and/or may receive an additional cancer therapy, such as surgery, radiation, chemotherapy, immunotherapy, hormone therapy, or a combination thereof.

### IV. Cells Generally

As outlined above, immune effector cells of the disclosure include at least NK cells, NKT cells, T cells, γδ T cells, macrophages, stem cells, Mucosal-associated invariant T (MAIT) cells, IL cells, Cytokine-induced killer (CIK) cells, or a mixture thereof. In some cases the immune effector cells, described herein, have been co-stimulated by one or more costimulatory agents as well as the particular costimulatory agent that itself is an artificial antigen presenting cell (which may be referred to as a non-natural cell that has antigen presenting cell activity). The immune effector cells may be subjected to TCR-stimulation and co-stimulation prior to their use in an individual and also prior to engineering them to produce the molecular adaptor or express one or more engineered antigen receptors.

In particular embodiments of the disclosure, the immune effector cells are derived from cells from the individual being treated (are autologous), although the cells may come from another one or more individuals (allogeneic).

In the context of expressing a heterologous nucleic acid sequence, a "host cell" can refer to a prokaryotic or eukaryotic cell, and it includes any transformable organism that is capable of replicating a vector and/or expressing a heterologous gene encoded by a vector. A host cell can, and has been, used as a recipient for vectors. A host cell may be "transfected," "transduced," "transformed," "electroporated," or subjected to any other process by which exogenous nucleic acid is transferred or introduced into the host cell. An engineered cell includes the primary subject cell and its progeny. As used herein, the terms "engineered" and "recombinant" cells or host cells are intended to refer to a cell into which an exogenous nucleic acid sequence, such as, for example, a vector, has been introduced. Therefore, recombinant cells are distinguishable from naturally occurring cells which do not contain a recombinantly introduced nucleic acid.

In certain embodiments, it is contemplated that RNAs or proteinaceous sequences may be co-expressed with other selected RNAs or proteinaceous sequences in the same host cell. Co-expression may be achieved by introducing two or more distinct recombinant vectors into the host cell. Alternatively, a single recombinant vector may be constructed to include multiple distinct coding regions for RNAs, which could then be expressed in host cells modified with the single vector.

Some vectors may employ control sequences that allow it to be replicated and/or expressed in both prokaryotic and eukaryotic cells. One of skill in the art would further understand the conditions under which to incubate all of the above described host cells to maintain them and to permit replication of a vector. Also understood and known are techniques and conditions that would allow large-scale production of vectors, as well as production of the nucleic acids encoded by vectors and their cognate polypeptides, proteins, or peptides.

The immune effector cells and host cells used in the disclosure are eukaryotic, including mammalian, although prokaryotic cells may be employed for manipulation in recombinant engineering of vectors or DNA to integrate into the vectors. The cells are particularly human, but can, *e.g.,* be associated with any animal of interest, particularly domesticated animals, such as equine, bovine, murine, ovine, canine, feline, *etc.* for use in their respective animal. In other embodiments, the cells are a cell line such as a commercially available cell line.

The immune effector cells can be autologous cells, syngeneic cells, allogenic cells and even in some cases, xenogeneic cells, such as in relation to the individual that is receiving the cells. The cells may be modified by changing the major histocompatibility complex ("MHC") profile, by inactivating β₂-microglobulin to reduce or prevent the formation of functional Class I MHC molecules, inactivation of Class II molecules, providing for expression of one or more MHC molecules, enhancing or inactivating cytotoxic capabilities by enhancing or inhibiting the expression of genes associated with the cytotoxic activity, or the like.

Expression vectors that encode an antigen receptor such as a CAR and/or molecular adaptor can be introduced into the cells as one or more DNA molecules or constructs, where there may be at least one marker that will allow for selection of host cells that contain the construct(s). The constructs can be prepared in conventional ways, where the genes and regulatory regions may be isolated, as appropriate, ligated, cloned in an appropriate cloning host, analyzed by restriction or sequencing, or other convenient means. Particularly, using PCR, individual fragments including all or portions of a functional unit may be isolated, where one or more mutations may be introduced using "primer repair", ligation, *in vitro* mutagenesis, *etc.,* as appropriate. The construct(s) once completed and demonstrated to have the appropriate sequences may then be introduced into the cell by any convenient means. The constructs may be integrated and packaged into non-replicating, defective viral genomes like Adenovirus, Adeno-associated virus (AAV), or Herpes simplex virus (HSV) or others, including retroviral or lentiviral vectors, for infection or transduction into cells. The constructs may include viral sequences for transfection, if desired. Alternatively, the construct may be introduced by fusion, electroporation, biolistics, transfection, lipofection, or the like. The host cells may be grown and expanded in culture before introduction of the construct(s), followed by the appropriate treatment for introduction of the construct(s) and integration of the construct(s). The cells are then expanded and screened by virtue of a marker present in the construct. Various markers that may be used successfully include hprt, neomycin resistance, thymidine kinase, hygromycin resistance, *etc.*

In many situations one may wish to be able to kill the modified cells, *e.g*.,where one wishes to terminate the treatment, the cells become neoplastic, in research where the absence of the cells after their presence is of interest, or other event. For this purpose one can provide for the expression of certain gene products in which one can kill the modified cells under controlled conditions. Suicide gene products, such as caspase 9, are examples of such products.

By way of illustration, cancer patients or patients susceptible to cancer or suspected of having cancer may be treated as follows. Cells modified as described herein may be administered to the patient and retained for extended periods of time. The individual may receive one or more administrations of the cells. The cell(s) would be modified and provided to the individual in need thereof.

### V. Polynucleotides

The present disclosure also encompasses a composition comprising a nucleic acid sequence encoding a molecular adaptor and an antigen receptor as defined herein and cells harboring the nucleic acid sequence(s). The nucleic acid molecule is a recombinant nucleic acid molecule, in particular aspects and may be synthetic. It may comprise DNA, RNA as well as PNA (peptide nucleic acid) or it may be a hybrid thereof.

Furthermore, it is envisaged for further purposes that nucleic acid molecules may contain, for example, thioester bonds and/or nucleotide analogues. The modifications may be useful for the stabilization of the nucleic acid molecule against endo- and/or exonucleases in the cell. The nucleic acid molecules may be transcribed by an appropriate vector comprising a chimeric gene that allows for the transcription of said nucleic acid molecule in the cell. In this respect, it is also to be understood that such polynucleotides can be used for "gene targeting" or "gene therapeutic" approaches. In another embodiment the nucleic acid molecules are labeled. Methods for the detection of nucleic acids are well known in the art, *e.g.,* Southern and Northern blotting, PCR or primer extension. This embodiment may be useful for screening methods for verifying successful introduction of the nucleic acid molecules described above during gene therapy approaches.

The nucleic acid molecule(s) may be a recombinantly produced chimeric nucleic acid molecule comprising any of the aforementioned nucleic acid molecules either alone or in combination. In specific aspects, the nucleic acid molecule is part of a vector.

The present disclosure therefore also relates to a composition comprising a vector comprising the nucleic acid molecule described in the present disclosure.

Many suitable vectors are known to those skilled in molecular biology, the choice of which would depend on the function desired and include plasmids, cosmids, viruses, bacteriophages and other vectors used conventionally in genetic engineering. Methods that are well known to those skilled in the art can be used to construct various plasmids and vectors; see, for example, the techniques described in Sambrook *et al.* (1989) and Ausubel, Current Protocols in Molecular Biology, Green Publishing Associates and Wiley Interscience, N.Y. (1989), (1994). Alternatively, the polynucleotides and vectors of the disclosure can be reconstituted into liposomes for delivery to target cells. A cloning vector may be used to isolate individual sequences of DNA. Relevant sequences can be transferred into expression vectors where expression of a particular polypeptide is required. Typical cloning vectors include pBluescript SK, pGEM, pUC9, pBR322 and pGBT9. Typical expression vectors include pTRE, pCAL-n-EK, pESP-1, pOP13CAT.

In specific embodiments, there is a vector that comprises a nucleic acid sequence that is a regulatory sequence operably linked to the nucleic acid sequence encoding an antigen-receptor, preferably a CAR or TCR, and/or molecular adaptor as defined herein. Such regulatory sequences (control elements) are known to the artisan and may include a promoter, a splice cassette, translation initiation codon, translation and insertion site for introducing an insert into the vector. In specific embodiments, the nucleic acid molecule is operatively linked to said expression control sequences allowing expression in eukaryotic or prokaryotic cells.

The term "regulatory sequence" refers to DNA sequences that are necessary to effect the expression of coding sequences to which they are ligated. The nature of such control sequences differs depending upon the host organism. In prokaryotes, control sequences generally include promoters, ribosomal binding sites, and terminators. In eukaryotes generally control sequences include promoters, terminators and, in some instances, enhancers, transactivators or transcription factors. The term "control sequence" is intended to include, at a minimum, all components the presence of which are necessary for expression, and may also include additional advantageous components.

The term "operably linked" refers to a juxtaposition wherein the components so described are in a relationship permitting them to function in their intended manner. A control sequence "operably linked" to a coding sequence is ligated in such a way that expression of the coding sequence is achieved under conditions compatible with the control sequences. In case the control sequence is a promoter, it is obvious for a skilled person that double-stranded nucleic acid is preferably used.

It is envisaged that in certain embodiments, a vector is an expression vector comprising the nucleic acid molecule encoding an antigen receptor as defined herein. In specific aspects, the vector is a viral vector, such as a lentiviral or retroviral vector. Lentiviral and retroviral vectors are commercially available, including from Clontech (Mountain View, CA) or GeneCopoeia (Rockville, MD), for example.

Regulatory elements for expression vectors ensuring expression in prokaryotes and/or eukaryotic cells are well known to those skilled in the art. In the case of eukaryotic cells they comprise normally promoters ensuring initiation of transcription and optionally poly-A signals ensuring termination of transcription and stabilization of the transcript. Possible regulatory elements permitting expression in prokaryotic host cells comprise, *e.g.,* the P_{L}, lac, trp or tac promoter in *E. coli,* and examples of regulatory elements permitting expression in eukaryotic host cells are the AOX1 or GAL1 promoter in yeast or the CMV-, SV40-, RSV-promoter (Rous sarcoma virus), CMV-enhancer, SV40-enhancer or a globin intron in mammalian and other animal cells.

Beside elements that are responsible for the initiation of transcription such regulatory elements may also comprise transcription termination signals, such as the SV40-poly-A site or the tk-poly-A site, downstream of the polynucleotide. Furthermore, depending on the expression system used leader sequences capable of directing the polypeptide to a cellular compartment or secreting it into the medium may be added to the coding sequence of the recited nucleic acid sequence and are well known in the art. The leader sequence(s) is (are) assembled in appropriate phase with translation, initiation and termination sequences, and preferably, a leader sequence capable of directing secretion of translated protein, or a portion thereof, into the periplasmic space or extracellular medium. Optionally, the heterologous sequence can encode a molecular adaptor including an N-terminal identification peptide imparting desired characteristics, *e.g.,* stabilization or simplified purification of expressed recombinant product; see supra. In this context, suitable expression vectors are known in the art such as Okayama-Berg cDNA expression vector pcDV1 (Pharmacia), pEF-Neo, pCDM8, pRc/CMV, pcDNA1, pcDNA3 (Invitrogen), pEF-DHFR and pEF-ADA, (Raum et al. Cancer Immunol Immunother (2001) 50(3), 141-150) or pSPORT1 (GIBCO BRL).

In some embodiments, the expression control sequences are eukaryotic promoter systems in vectors capable of transforming of transfecting eukaryotic host cells, but control sequences for prokaryotic hosts may also be used. Once the vector has been incorporated into the appropriate host, the host is maintained under conditions suitable for high level expression of the nucleotide sequences, and as desired, the collection and purification of the polypeptide of the disclosure may follow. In particular embodiments, one or more encodable sequences are regulated by expression control sequences that are responsive to hypoxic environments.

Additional regulatory elements may include transcriptional as well as translational enhancers. Advantageously, the above-described vectors of the disclosure comprises a selectable and/or scorable marker. Selectable marker genes useful for the selection of transformed cells are well known to those skilled in the art and comprise, for example, antimetabolite resistance as the basis of selection for dhfr, which confers resistance to methotrexate (Reiss, Plant Physiol. (Life-Sci. Adv.) 13 (1994), 143-149); npt, which confers resistance to the aminoglycosides neomycin, kanamycin and paromycin (Herrera-Estrella, EMBO J. 2 (1983), 987-995) and hygro, which confers resistance to hygromycin (Marsh, Gene 32 (1984), 481-485). Additional selectable genes have been described, namely trpB, which allows cells to utilize indole in place of tryptophan; hisD, which allows cells to utilize histinol in place of histidine (Hartman, Proc. Natl. Acad. Sci. USA 85 (1988), 8047); mannose-6-phosphate isomerase which allows cells to utilize mannose (WO 94/20627) and ODC (ornithine decarboxylase) which confers resistance to the ornithine decarboxylase inhibitor, 2-(difluoromethyl)-DL-ornithine, DFMO (McConlogue, 1987, In: Current Communications in Molecular Biology, Cold Spring Harbor Laboratory ed.) or deaminase from Aspergillus terreus which confers resistance to Blasticidin S (Tamura, Biosci. Biotechnol. Biochem. 59 (1995), 2336-2338).

Useful scorable markers are also known to those skilled in the art and are commercially available. Advantageously, said marker is a gene encoding luciferase (Giacomin, Pl. Sci. 116 (1996), 59-72; Scikantha, J. Bact. 178 (1996), 121), green fluorescent protein (Gerdes, FEBS Lett. 389 (1996), 44-47) or β-glucuronidase (Jefferson, EMBO J. 6 (1987), 3901-3907). This embodiment is particularly useful for simple and rapid screening of cells, tissues and organisms containing a recited vector.

As described above, the recited nucleic acid molecule can be used in a cell, alone, or as part of a vector to express the encoded polypeptide in cells. The nucleic acid molecules or vectors containing the DNA or RNA sequence(s) encoding any one of the specific antigen receptor and/or molecular adaptor constructs is introduced into the cells that in turn produce the polypeptide of interest. The recited nucleic acid molecules and vectors may be designed for direct introduction or for introduction *via* liposomes, or viral vectors (*e.g.,* adenoviral, retroviral) into a cell.

In accordance with the above, the present disclosure relates to methods to derive vectors, particularly plasmids, cosmids, viruses and bacteriophages used conventionally in genetic engineering that comprise a nucleic acid molecule encoding the polypeptide sequence of the constructs defined herein. In at least some cases, the vector is an expression vector and/or a gene transfer or targeting vector. Expression vectors derived from viruses such as retroviruses, lentiviruses, vaccinia virus, adeno-associated virus, herpes viruses, or bovine papilloma virus, may be used for delivery of the recited polynucleotides or vector into targeted cell populations. Methods which are well known to those skilled in the art can be used to construct recombinant vectors; see, for example, the techniques described in Sambrook *et al.* (*loc cit.*), Ausubel (1989, *loc cit*.) or other standard text books. Alternatively, the recited nucleic acid molecules and vectors can be reconstituted into liposomes for delivery to target cells. The vectors containing the nucleic acid molecules of the disclosure can be transferred into the host cell by well-known methods, which vary depending on the type of cellular host. For example, calcium chloride transfection is commonly utilized for prokaryotic cells, whereas calcium phosphate treatment or electroporation may be used for other cellular hosts; see Sambrook, *supra.*

### VI.Pharmaceutical Compositions

In accordance with this disclosure, the term "pharmaceutical composition" relates to a composition for administration to an individual. In specific aspects of the disclosure, the pharmaceutical composition comprises a plurality of immune effector cells as described herein. In certain aspects of the disclosure, the pharmaceutical composition comprises a purified molecular adaptor as described herein. In a particular embodiment, the pharmaceutical composition comprises a composition for parenteral, transdermal, intraluminal, intra-arterial, intrathecal or intravenous administration or for direct injection into a cancer. It is in particular envisaged that the pharmaceutical composition is administered to the individual *via* infusion or injection. Administration of the suitable compositions may be effected by different ways, *e.g.,* by intravenous, subcutaneous, intraperitoneal, intramuscular, topical and/or intradermal administration. The immune effector cells may be administered to the individual systemically or locally.

The pharmaceutical composition of the present disclosure may further comprise a pharmaceutically acceptable carrier. Examples of suitable pharmaceutical carriers are well known in the art and include phosphate buffered saline solutions, water, emulsions, such as oil/water emulsions, various types of wetting agents, sterile solutions, cryopreservation agents,, *etc.* Compositions comprising such carriers can be formulated by well-known conventional methods. These pharmaceutical compositions can be administered to the subject at a suitable dose.

The dosage regimen will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently. Progress can be monitored by periodic assessment. The immune effector cells encompassed by the disclosure may be administered *via* intravenous infusion. Doses of the immune effector cells described herein can range from 1x10⁶/m² to 2x10⁸/m², and in specific embodiments up to 10¹⁰ cells may be utilized. In specific cases, suitable doses for a therapeutic effect would be between about 10⁶ and about 10⁹ cells per dose, and they may be in a series of dosing cycles. A particular dosing regimen comprises four one-week dosing cycles of escalating doses, starting at about 10⁷ cells on Day 0, increasing incrementally up to a target dose of about 10⁸ cells by Day 5. Suitable modes of administration include intravenous, subcutaneous, intra-cavitary (for example by reservoir-access device), intraperitoneal, and direct injection into a tumor mass, as examples only. The amounts of each active agent included in the compositions described herein (*e.g.,* the amount per each cell to be contacted or the amount per certain body weight) can vary in different applications. In general, the concentration of transduced immune effector cells desirably should be sufficient to provide in the subject being treated at least from about 1×10⁶ to about 1×10⁹ transduced immune effector cells, including from about 1×10⁷ to about 5×10⁸ transduced immune effector cells, although any suitable amount can be utilized either above, *e.g.,* greater than 5×10⁸ cells, or below, *e.g.,* less than 1×10⁷ cells. The dosing schedule can be based on well-established cell-based therapies or an alternate continuous infusion strategy can be employed.

The compositions of the disclosure may be administered locally or systemically. Administration will generally be parenteral, e.g., intravenous; DNA may also be administered directly to the target site, *e.g.,* by biolistic delivery to an internal or external target site or by catheter to a site in an artery. In a preferred embodiment, the pharmaceutical composition is administered subcutaneously and in an even more preferred embodiment intravenously. Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishes, electrolyte replenishers (such as those based on Ringer's dextrose), and the like. Preservatives and other additives may also be present such as, for example, antimicrobials, anti-oxidants, chelating agents, cryopreservation agents, and inert gases and the like. In addition, the pharmaceutical composition of the present disclosure might comprise proteinaceous carriers, like, *e.g.,* serum albumin or immunoglobulin, preferably of human origin. It is envisaged that the pharmaceutical composition of the disclosure might comprise, in addition to the CAR constructs or nucleic acid molecules or vectors encoding the same (as described in this disclosure), further biologically active agents, depending on the intended use of the pharmaceutical composition.

### VII. Kits of the Disclosure

Any of the immune effector cell compositions and/or reagent(s) to produce same that are described herein may be comprised in a kit. In a non-limiting example, cells or reagents to manipulate cells may be comprised in a kit. In certain embodiments, NKT cells or a population of cells that comprises NKT cells may be comprised in a kit. Such a kit may or may not have one or more reagents for manipulation of cells. Such reagents include small molecules, proteins, nucleic acids, antibodies, buffers, primers, nucleotides, salts, and/or a combination thereof, for example. Nucleic acids that encode one or more cytokines, or cytokines themselves, may be included in the kit. Proteins, such as cytokines or antibodies, including agonistic monoclonal antibodies, may be included in the kit. Substrates that comprise the antibodies, or naked substrates themselves, may be included in the kit, and in some embodiments reagents to generate antibody-bearing substrates are included in the kit. The substrates may be of any kind including a bead or plate. Cells that comprise antigen presenting cell activity or reagents to generate same may be included in the kit. Nucleic acids that encode chimeric antigen receptors (CARs) and/or T-cell receptors may be included in the kit, including reagents to generate same.

In particular aspects, the kit comprises immune effector cells and/or purified molecular adaptors as described herein and also another cancer therapy. In some cases, the kit, in addition to the cell therapy embodiments, also includes a second cancer therapy, such as chemotherapy, hormone therapy, and/or immunotherapy, such as a checkpoint inhibitor for example. The kit(s) may be tailored to a particular cancer for an individual and comprise respective second cancer therapies for the individual.

The kits may comprise suitably aliquoted compositions of the present invention. The components of the kits may be packaged either in aqueous media or in lyophilized form. The container means of the kits will generally include at least one vial, test tube, flask, bottle, syringe or other container means, into which a component may be placed, and preferably, suitably aliquoted. Where there are more than one component in the kit, the kit also may generally contain a second, third or other additional container into which the additional components may be separately placed. However, various combinations of components may be comprised in a vial. The kits of the present invention also will typically include a means for containing the composition and any other reagent containers in close confinement for commercial sale. Such containers may include injection or blow-molded plastic containers into which the desired vials are retained.

When the components of the kit are provided in one and/or more liquid solutions, the liquid solution is an aqueous solution, with a sterile aqueous solution being particularly preferred. In which case, the container means may itself be a bag, syringe, pipette, and/or other such like apparatus, from which the formulation may be applied to an affected area of the body, injected into an animal, and/or even applied to and/or mixed with the other components of the kit. When immune effector cells are a component of the kit, they may be provided in frozen form, with one or more cryopreservation agents added. In which case, the kit may comprise component(s) for thawing the frozen cells in a manner that maintains cell viability. However, the components of the kit or parts thereof may be provided as dried powder(s). When reagents and/or components are provided as a dry powder, the powder can be reconstituted by the addition of a suitable solvent. It is envisioned that the solvent may also be provided in another container means.

### VIII.Combination Therapy

In certain embodiments of the invention, methods of the present disclosure for clinical aspects are combined with other agents effective in the treatment of hyperproliferative disease, such as anti-cancer agents. An "anti-cancer" agent is capable of negatively affecting cancer in a subject, for example, by killing cancer cells, inducing apoptosis in cancer cells, reducing the growth rate of cancer cells, reducing the incidence or number of metastases, reducing tumor size, inhibiting tumor growth, reducing the blood supply to a tumor or cancer cells, promoting an immune response against cancer cells or a tumor, preventing or inhibiting the progression of cancer, or increasing the lifespan of a subject with cancer. More generally, these other compositions would be provided in a combined amount effective to kill or inhibit proliferation of the cell. This process may involve contacting the cancer cells with the expression construct and the agent(s) or multiple factor(s) at the same time. This may be achieved by contacting the cell with a single composition or pharmacological formulation that includes both agents, or by contacting the cell with two distinct compositions or formulations, at the same time, wherein one composition includes the expression construct and the other includes the second agent(s).

Tumor cell resistance to chemotherapy and radiotherapy agents represents a major problem in clinical oncology. One goal of current cancer research is to find ways to improve the efficacy of chemo- and radiotherapy by combining it with cell therapy. In the context of the present invention, it is contemplated that cell therapy could be used similarly in conjunction with chemotherapeutic, radiotherapeutic, or immunotherapeutic intervention, for example.

The present disclosed therapy may precede or follow the other agent treatment by intervals ranging from minutes to weeks. In embodiments where the other agent and present methods are applied separately to the individual, one would generally ensure that a significant period of time did not expire between the time of each delivery, such that the agent and inventive therapy would still be able to exert an advantageously combined effect on the cell. In such instances, it is contemplated that one may contact the cell with both modalities within about 12-24 h of each other and, in at least some cases, within about 6-12 h of each other. In some situations, it may be desirable to extend the time period for treatment significantly, however, where several days (2, 3, 4, 5, 6 or 7) to several weeks (1, 2, 3, 4, 5, 6, 7 or 8) lapse between the respective administrations. It is expected that the treatment cycles would be repeated as necessary. It also is contemplated that various standard therapies, as well as surgical intervention, may be applied in combination with the inventive cell therapy.

### A. Chemotherapy

In some embodiments, the methods of the disclosure are employed with, before, or after chemotherapy. The chemotherapy may be of any kind. In specific embodiments, the chemotherapy comprises Adriamycin PFS (Doxorubicin Hydrochloride); Adriamycin RDF (Doxorubicin Hydrochloride); Clafen (Cyclophosphamide); Cyclophosphamide; Cytoxan (Cyclophosphamide); Doxorubicin Hydrochloride; Neosar (Cyclophosphamide); Vincasar PFS (Vincristine Sulfate); and/or Vincristine Sulfates. Cancer therapies also include a variety of combination therapies with both chemical and radiation-based treatments. Combination chemotherapies include, for example, abraxane, altretamine, docetaxel, herceptin, methotrexate, novantrone, zoladex, cisplatin (CDDP), carboplatin, procarbazine, mechlorethamine, cyclophosphamide, camptothecin, ifosfamide, melphalan, chlorambucil, busulfan, nitrosurea, dactinomycin, daunorubicin, doxorubicin, bleomycin, plicomycin, mitomycin, etoposide (VP16), tamoxifen, raloxifene, estrogen receptor binding agents, taxol, gemcitabien, navelbine, farnesyl-protein tansferase inhibitors, transplatinum, 5-fluorouracil, vincristin, vinblastin and methotrexate, or any analog or derivative variant of the foregoing.

### B. Radiotherapy

Other factors that cause DNA damage and have been used extensively include what are commonly known as γ-rays, X-rays, proton beams, electron beams, and/or the directed delivery of radioisotopes to tumor cells. Other forms of DNA damaging factors are also contemplated such as microwaves and UV-irradiation. It is most likely that all of these factors effect a broad range of damage on DNA, on the precursors of DNA, on the replication and repair of DNA, and on the assembly and maintenance of chromosomes.

The terms "contacted" and "exposed," when applied to a cell, are used herein to describe the process by which a therapeutic construct and a chemotherapeutic or radiotherapeutic agent are delivered to a target cell or are placed in direct juxtaposition with the target cell. To achieve cell killing or stasis, both agents are delivered to a cell in a combined amount effective to kill the cell or prevent it from dividing.

### C. Immunotherapy

Immunotherapeutics generally rely on the use of immune effector cells and molecules to target and destroy cancer cells and in this specific embodiment the immunotherapeutics are other than those of the present disclosure The immune effector may be, for example, an antibody specific for some marker on the surface of a tumor cell. The antibody alone may serve as an effector of therapy or it may recruit other cells to actually effect cell killing. The antibody may be a checkpoint inhibitor, such as ipilimumab, nivolumab, or pembrolizumab, for example. The antibody also may be conjugated to a drug or toxin (chemotherapeutic, radionuclide, ricin A chain, cholera toxin, pertussis toxin, *etc*.) and serve merely as a targeting agent. Alternatively, a non-antibody molecule may be used to modulate the immune system. Alternatively, the effector may be a lymphocyte carrying a surface molecule that interacts, either directly or indirectly, with a tumor cell target. Various immune effector cells include cytotoxic T cells and NK cells. In preferred embodiments, the immune effector cells are or comprise NKT cells.

Immunotherapy could thus be used as part of a combined therapy, in conjunction with the present cell therapy. The general approach for combined therapy is generally such that the tumor cell bears some marker that is amenable to targeting, *i.e.,* is not present on some or the majority of other cells. Many tumor markers exist and any of these may be suitable for targeting in the context of immunotherapeutics to be used with those of the present disclosure. Common tumor markers include carcinoembryonic antigen, prostate specific antigen, urinary tumor associated antigen, fetal antigen, tyrosinase (p97), gp68, TAG-72, HMFG, Sialyl Lewis Antigen, MucA, MucB, PLAP, estrogen receptor, laminin receptor, erb B and p155.

### D. Genes

In yet another embodiment, the secondary treatment is a gene therapy in which a therapeutic polynucleotide is administered before, after, or at the same time as the present invention clinical embodiments. A variety of expression products are encompassed within the invention, including inducers of cellular proliferation, inhibitors of cellular proliferation, or regulators of programmed cell death.

### E. Surgery

Approximately 60% of persons with cancer will undergo surgery of some type, which includes preventative, diagnostic or staging, curative and palliative surgery. Curative surgery is a cancer treatment that may be used in conjunction with other therapies, such as the treatment of the present invention, chemotherapy, radiotherapy, hormonal therapy, gene therapy, immunotherapy and/or alternative therapies.

Surgery includes resection in which all or part of cancerous tissue is physically removed, excised, and/or destroyed. Tumor resection refers to physical removal of at least part of a tumor. In addition to tumor resection, treatment by surgery includes laser surgery, cryosurgery, electrosurgery, and microscopically controlled surgery (Mohs' surgery). It is further contemplated that the present invention may be used in conjunction with removal of superficial cancers, precancers, or incidental amounts of normal tissue.

Upon excision of part or all of cancerous cells, tissue, or tumor, a cavity may be formed in the body. Treatment may be accomplished by perfusion, direct injection or local application of the area with an additional anti-cancer therapy. Such treatment may be repeated, for example, every 1, 2, 3, 4, 5, 6, or 7 days, or every 1, 2, 3, 4, and 5 weeks or every 1, 2, 3, 4, 5, 6, 7, 8, 9, 10, 11, or 12 months. These treatments may be of varying dosages as well.

### F. Other agents

It is contemplated that other agents may be used in combination with the present methods and compositions to improve the therapeutic efficacy of treatment. These additional agents include immunomodulatory agents, agents that affect the upregulation of cell surface receptors and GAP junctions, cytostatic and differentiation agents, inhibitors of cell adhesion, or agents that increase the sensitivity of the hyperproliferative cells to apoptotic inducers. Immunomodulatory agents include tumor necrosis factor; interferon alpha, beta, and gamma; IL-2 and other cytokines; F42K and other cytokine analogs; or MIP-1, MIP-1beta, MCP-1, RANTES, and other chemokines. It is further contemplated that the upregulation of cell surface receptors or their ligands such as Fas / Fas ligand, DR4 or DR5 / TRAIL would potentiate the apoptotis inducing of the cells or compositions of the present disclosure by establishment of an autocrine or paracrine effect on hyperproliferative cells. Increased intercellular signaling by elevating the number of GAP junctions may increase the anti-hyperproliferative effects on the neighboring hyperproliferative cell population. In other embodiments, cytostatic or differentiation agents can be used in combination with the present invention to improve the anti-hyerproliferative efficacy of the treatments. Inhibitors of cell adhesion are contemplated to improve the efficacy of the present invention. Examples of cell adhesion inhibitors are focal adhesion kinase (FAKs) inhibitors and Lovastatin. It is further contemplated that other agents that increase the sensitivity of a hyperproliferative cell to apoptosis, such as the antibody c225, could be used in combination with the present invention to improve the treatment efficacy.

### EXAMPLES

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

### EXAMPLE 1

### DESIGN AND TESTING OF EXAMPLES OF MOLECULAR ADAPTORS

To test particular embodiments of the disclosure, retroviral vectors were constructed expressing a 1) CAR19 consisting of anti-CD 19 single-chain variable fragment (scFv) derived from a murine monoclonal antibody FMC-63, connected *via* a short spacer and transmembrane domain from human CD8, the CD28 costimulatory domain, and the CD3ζ T-cell activation domain; 2) a molecular adaptor construct encoding a truncated extracellular domain of CD19 (CD19 extracellular), a scFv derived from 14g2a mAb specific for GD2 ganglioside (a-GD2), with or without human IL-15 (IL15), human IL-15Ra (IL15Ra), or IL15RA-IL15 complex. IL-15 or IL15RA-IL15 were expressed as fusion proteins with CD19 extracellular (*e.g.* a-GD1-CD19-IL15) or as separately secreted proteins (*e.g*. a-GD2-CD19.IL15). An anti-GD2 CAR with 14g2a scFv, CD28 costimulatory domain, and the CD3ζ T-cell activation domain (CARGD2) served as a positive control (FIG. 1). A signal peptide sequence derived from the human IgG heavy chain was included at the beginning of the FMC63 and 14g2a scFvs to ensure that expressed CAR proteins were inserted into the cell membrane and that expressed molecular adaptor proteins were secreted from the cell. One skilled in the art will appreciate that other signal peptide sequences could be used.

### EXAMPLE 2

### EXPRESSION OF MOLECULAR ADAPTORS IN NKTS AND TESTING IN VITRO FUNCTIONAL ACTIVITY

Retroviral transduction of human NKT cells with the molecular adaptor constructs resulted in effective expression of CAR19 on the cell surface (FIG. 2A) and secretion of such adaptors. CD19-negative/GD2-positive CHLA-255 neuroblastoma cells became CD19-positive after treatment with the supernatant from lymphocytes transduced with a-GD2-CD19 molecular adaptor, as measured by flow cytometry. Supernatant from lymphocytes transduced with a-GD2 only (without CD19 extracellular) did not cause CHLA-255 cells to become CD19-positive. CD19-negative/GD2-negative LAN-6 neuroblastoma cells remained CD19-negative after the same treatments (FIG. 2B). CAR19 NKT cells, expressing all examined molecular adaptor constructs that include CD19 extracellular demonstrated a dose-dependent in vitro cytotoxicity against CHLA-255 neuroblastoma cells that was comparable to cytotoxicity mediated by NKT cells expressing CARGD2 as a positive control. In contrast, a molecular adaptor negative control without CD19 domain (CAR19.a-GD2) failed to induce CAR19 NKT cell cytotoxicity against CHLA-255 cells (FIG. 3).

### EXAMPLE 3

### TESTING IN VIVO ANTI-TUMOR ACTIVITY OF NKT CELLS EXPRESSING MOLECULAR ADAPTOR CONSTRUCTS

To test *in vivo* antitumor activity of NKT cells expressing the molecular adaptor constructs we used a previously described model of metastatic neuroblastoma in NSG mice (9). Mice were injected with 10⁶ CHLA-255/luc cells and 7 days later treated with 10⁷ NKT cells expressing constructs described in FIG. 1. NKT cells expressing only CAR19.a-GD2 or CARGD2 were used as a negative or a positive control, respectively. Antitumor activity was monitored by weekly bioluminescent imaging. FIG. 4 demonstrates that NKT cells expressing CAR19.a-GD2-CD19-IL15-IL15Ra mediated potent anti-tumor activity achieving median survival about 80 days, similar to the survival of mice in the CARGD2 positive control group. In contrast, all other constructs produced minimal anti-tumor activity, extending survival only by one week compared to negative control. These results exemplify an embodiment as shown schematically in FIG. 12 and FIG. 13C.

### EXAMPLE 4

### GENERATION OF ADDITIONAL MOLECULAR ADAPTOR CONSTRUCTS BASED ON THE LEAD CANDIDATE DESIGN

The distinct features of the therapeutically active construct (CAR19.a-GD2-CD19-IL15-IL15Ra) compared with non-active analogs are the following: 1) both IL15 and IL15Ra are present instead of only IL-15 or only IL-15Ra; 2) IL15 and IL15Ra are fused to CD19 and therefore presented to CAR19 NKTs on the tumor cell surface in close molecular proximity to CD19 (the target antigen) instead of being presented to CAR19 NKTs in soluble forms. To further explore the critical requirements for the molecular adaptor activity we asked how the activity of the lead construct would be affected by: 1) reversal of the direction of IL-15 to IL-15R fusion; 2) leaving IL-15Ra fused to CD19 while making IL-15 secreted as a soluble protein. To address these questions we design the following constructs: CAR19.a-GD2-CD19-IL15Ra, CAR19.a-GD2-CD19-IL15RA.IL15, CAR19.a-GD2-CD19-IL15Ra-IL15 (FIG. 5).

### EXAMPLE 5

### EXPRESSION AND IN VITRO FUNCTIONAL ACTIVITY OF NKT CELLS EXPRESSING ANALOGUES OF THE LEAD MOLECULAR ADAPTOR

Following generation of the constructs described in FIG. 5, their expression was tested in NKT cells and *in vitro* cytotoxicity testing was performed using GD2-positive and GD2-negative neuroblastoma cell lines as targets (FIG. 6). The new constructs can be effectively expressed in NKTs (FIG. 6A) and mediate a similar level of GD2-specific cytotoxicity as the original lead construct (FIG. 6B).

### EXAMPLE 6

### TESTING IN VIVO ANT-TUMOR ACTIVITY OF NKT CELLS EXPRESSING ANALOGUES OF THE LEAD MOLECULAR ADAPTOR

To test *in vivo* antitumor activity of NKT cells expressing the lead molecular adaptor and new analogues, the *in vivo* therapeutic experiment using the same model as described in FIG. 4 was performed. The original lead construct reproducibly mediated potent therapeutic activity (FIG. 7). In contrast, none of the new analogues showed a significant anti-tumor activity. These results indicate that at least in some embodiments, it is preferred to have both IL-15 and IL-15Ra fused in one defined orientation and co-presented with the target antigen on the tumor cell surface. However, one skilled in the art will recognize that different arrangements of components may be useful for different molecular adaptors, and will appreciate that particular arrangements can be determined through ordinary experimentation.

### EXAMPLE 7

### ENGINEERED TRANS-PRESENTATION OF IL-15 ENHANCES THERAPEUTIC EFFICACY OF GD2-SPECIFIC CAR NKT CELLS IN A XENOGENIC MODEL OF NEUROBLASTOMA

Vα24-invariant natural killer T cells (NKTs) are an attractive effector for expression of tumor-specific chimeric antigen receptors (CARs) because of their natural anti-tumor properties and ability to preferentially localize to the tumor site in neuroblastoma (NB) and other solid tumors. It was previously demonstrated that adoptively transferred human NKTs expressing GD2-specific CARs more effectively localize to the tumor site than T cells and mediate anti-tumor activity in a xenogenic model of NB in NOD/SCID/IL-2Rγ^{null} (NSG) mice. It was determined that NKTs can be inhibited within the tumor microenvironment (TME) and partially rescued by engineering them to express IL-15. These findings led to development of therapeutic NKTs co-expressing CARGD2 and IL-15, which are currently being tested in a phase 1 clinical trial (NCT03294954). Next, it was considered that the therapeutic potency of NKTs expressing CARGD2 and IL-15 can be further enhanced if IL-15 is engineered to be presented *in trans,* mimicking the physiological mode of IL-15 presentation in complex with IL-15Ra on adjacent cells. To that end, NKTs were transduced with retroviral constructs encoding CARGD2 for cell surface expression, and they secreted forms of IL-15, IL-15Ra, an IL15-IL15Ra fusion, or IL-15 with IL-15Ra as separately secreted proteins. In another set of constructs, IL-15Ra or IL15-IL15Ra were linked to a single-chain variable fragment (scFv) targeting one of the following antigens, all highly expressed on NB cells: GD2, CD24, or CD56. Co-expression of cell surface CARGD2 and the described secreted molecules did not impact CAR expression in NKTs (FIG. 10) or their *in vitro* cytotoxicity against GD2-positive NB cells. However, NKTs co-expressing CARGD2 and fusions of the IL15-IL15Ra complex with the anti-CD24 or anti-CD56 scFvs significantly increased long-term survival of NSG mice with metastatic NB xenografts compared with CARGD2.IL15 NKTs (FIG. 11). These findings reveal that dual targeting of tumor cells with a CAR specific for one antigen and an IL15-IL15Ra-scFv complex specific for another antigen, as shown schematically in FIG. 13B, overcomes the suppressive TME and activates NKTs for effective tumor elimination. Furthermore, NKTs co-expressing CARGD2, IL-15, and a molecular adaptor that also targets GD2 also provided significantly increased survival compared to CARGD2.IL15 NKTs (FIG. 11). These findings reveal that tumor cells can be targeted with a CAR and a molecular adapter that recognize the same antigen, as shown schematically in FIG. 13A. These novel embodiments of immune engineering are broadly applicable to other therapeutic effector cells as well as other types of solid tumors.

### EXAMPLES OF SEQUENCES

CAR19: 
MEFGLSWLFLVAILKGVQCSRDIQMTQTTSSLSASLGDRVTISCRASQDISKYLNWYQQK
PDGTVKLLIYHTSRLHSGVPSRFSGSGSGTDYSLTISNLEQEDIATYFCQQGNTLPYTFG
GGTKLELKRGGGGSGGGGSGGGGSGGGGSEVQLQQSGPGLVAPSQSLSVTCTVSGVSLPD
YGVSWIRQPPRKGLEWLGVIWGSETTYYNSALKSRLTIIKDNSKSQVFLKMNSLQTDDTA
IYYCAKHYYYGGSYAMDYWGQGTTVTVSSTTTPAPRPPTPAPTIASQPLSLRPEACRPAA
GGAVHTRGLDFACDIYIWAPLAGTCGVLLLSLVITLYCRSKRSRLLHSDYMNMTPRRPGP
TRKHYQPYAPPRDFAAYRSRVKFSRSADAPAYQQGQNQLYNELNLGRREEYDVLDKRRGR
DPEMGGKPRRKNPQEGLYNELQKDKMAEAYSEIGMKGERRRGKGHDGLYQGLSTATKDTY
DALHMQALPPR (SEQ ID NO:1)
CARGD2: 
MEFGLSWLFLVAILKGVQCSRDILLTQTPLSLPVSLGDQASISCRSSQSLVHRNGNTYLH
WYLQKPGQSPKLLIHKVSNRFSGVPDRFSGSGSGTDFTLKISRVEAEDLGVYFCSQSTHV
PPLTFGAGTKLELKRADAAPTVSIFPGSGGGGSGGEVKLQQSGPSLVEPGASVMISCKAS
GSSFTGYNMNWVRQNIGKSLEWIGAIDPYYGGTSYNQKFKGRATLTVDKSSSTAYMHLKS
LTSEDSAVYYCVSGMEYWGQGTSVTVSSTRTTTPAPRPPTPAPTIASQPLSLRPEACRPA
AGGAVHTRGLDFACDIYIWAPLAGTCGVLLLSLVITLYCRSKRSRLLHSDYMNMTPRRPG
PTRKHYQPYAPPRDFAAYRSRVKFSRSADAPAYQQGQNQLYNELNLGRREEYDVLDKRRG
RDPEMGGKPRRKNPQEGLYNELQKDKMAEAYSEIGMKGERRRGKGHDGLYQGLSTATKDT
YDALHMQALPPR (SEQ ID NO:2)
IgG4 hinge:
   ESKYGPPCPSCP (SEQ ID NO:3)
CD19 extracellular: 
PEEPLVVKVEEGDNAVLQCLKGTSDGPTQQLTWSRESPLKPFLKLSLGLPGLGIHMRPLAIWL
FIFNVSQQMGGFYLCQPGPPSEKAWQPGWTVNVEGSGELFRWNVSDLGGLGCGLKNRSSE
GPSSPSGKLMSPKLYVWAKDRPEIWEGEPPCLPPRDSLNQSLSQDLTMAPGSTLWLSCGV
PPDSVSRGPLSWTHVHPKGPKSLLSLELKDDRPARDMWVMETGLLLPRATAQDAGKYYCH
RGNLTMSFHLEITARPVLWHWLLRTGGWK (SEQ ID NO:4)
T2A:
   RAEGRGSLLTCGDVEENPGP (SEQ ID NO:5)
P2A:
   ATNFSLLKQAGDVEENPGP (SEQ ID NO:6)
Linker 1:
   GGGGSGGGGSGGGSLQGTTC (SEQ ID NO:7)
Linker 2:
   SGGGSGGGGSGGGGSGGGGSGGGSLQ (SEQ ID NO:8)
IL15: 
MRISKPHLRSISIQCYLCLLLNSHFLTEAGIHVFILGCFSAGLPKTEANWVNVISDLKKIEDLI
QSMHIDATLYTESDVHPSCKVTAMKCFLLELQVISLESGDASIHDTVENLIILANNSLSSNGNV
TESGCKECEELEEKNIKEFLQSFVHIVQMFINTS (SEQ ID NO:9)
IL15Ra extracellular domain: 
MAPRRARGCRTLGLPALLLLLLLRPPATRGITCPPPMSVEHADIWVKSYSLYSRERYICNSGFK
RKAGTSSLTECVLNKATNVAHWTTPSLKCIRDPALVHQRPAPPSTVTTAGVTPQPESLSPSGKE
PAASSPSSNNTAATTAAIVPGSQLMPSKSPSTGTTEISSHESSHGTPSQTTAKNWELTASASHQ
PPGVYPQGHSDTT (SEQ ID NO:10)
Anti-CD24 scFv: 
MEFGLSWLFLVAILKGVQCDVHLQESGPDLVKPSQSLSLTCTVTGYSITSGYTWHWIRQFPGNT
VEWMetGYIQYTGSTRYNPALRGRLSISRDTSKNQFFLQLISVTTADTGTYFCARGTTASFDYW
GQGTTLTVASAGSAGGGGSGGGGSGGGGSDIVMSQSPSSLNVSVGEKVTMRCRSSQSLLYSSDQ
KNYLTWYQQKPGQSPKLLISWASTRASGVPDRFTGSGSGTDFTLTISSVKAEDLGVYYCQQYFI
YPLTFGVGTKLGLKAAA (SEQ ID NO:11)
Anti-CD56 scFv: 
MEFGLSWLFLVAILKGVQCSRQVQLVESGGGVVQPGRSLRLSCAASGFTFSSFGMHWVRQAPGK
GLEWVAYISSGSFTIYYADSVKGRFTISRDNSKNTLYLQMNSLRSEDTAMYYCARMRKGYAMDY
WGQGTLVTVSSGGGGSGGGGSGGGGSGGGGSDVLMTQTPLSLPVTLGQPASISCRSSQIIIHSD
GNTYLEWFQQRPGQSPRLLIYKVSNRFSGVPDRFSGSGSGTDFTLKISRVEAEDVGVYYCFQGS
HVPHTFGGGTKVEIKP (SEQ ID NO:12)
14g2a scFv: 
MEFGLSWLFLVAILKGVQCSRDILLTQTPLSLPVSLGDQASISCRSSQSLVHRNGNTYLH
WYLQKPGQSPKLLIHKVSNRFSGVPDRFSGSGSGTDFTLKISRVEAEDLGVYFCSQSTHV
 
PPLTFGAGTKLELKRADAAPTVSIFPGSGGGGSGGEVKLQQSGPSLVEPGASVMISCKAS
GSSFTGYNMNWVRQNIGKSLEWIGAIDPYYGGTSYNQKFKGRATLTVDKSSSTAYMHLKS
LTSEDSAVYYCVSGMEYWGQGTSVTVSSAKTTPPSVYGRVTVSSA (SEQ ID NO:13)
E2A:
   QCTNYALLKLAGDVESNPGP (SEQ ID NO:14)

### REFERENCES

1. Kalos M, June CH. Adoptive T cell transfer for cancer immunotherapy in the era of synthetic biology. Immunity 2013;39(1):49-60.
2. Dotti G, Gottschalk S, Savoldo B, Brenner MK. Design and development of therapies using chimeric antigen receptor-expressing T cells. Immunol.Rev. 2014;257(1):107-126.
3. Scarfo I, Maus MV. Current approaches to increase CAR T cell potency in solid tumors: targeting the tumor microenvironment. J Immunother Cancer 2017;5:28.
4. Pule MA, et al. Virus-specific T cells engineered to coexpress tumor-specific receptors: persistence and antitumor activity in individuals with neuroblastoma. Nat.Med. 2008;14(11): 1264-1270.
5. Louis CU, et al. Antitumor activity and long-term fate of chimeric antigen receptorpositive T cells in patients with neuroblastoma. Blood 2011;118(23):6050-6056.
6. Pegram HJ, Smith EL, Rafiq S, Brentjens RJ. CAR therapy for hematological cancers: can success seen in the treatment of B-cell acute lymphoblastic leukemia be applied to other hematological malignancies? Immunotherapy. 2015;7(5):545-561.
7. Heczey A, et al. Invariant NKT cells with chimeric antigen receptor provide a novel platform for safe and effective cancer immunotherapy. Blood 2014;124(18):2824-2833.
8. Song L, et al. Valpha24-invariant NKT cells mediate antitumor activity via killing of tumor-associated macrophages. J.Clin.Invest 2009;119(6):1524-1536.
9. Liu D, et al. IL-15 protects NKT cells from inhibition by tumor-associated macrophages and enhances antimetastatic activity. J.Clin.Invest 2012;122:2221-2233.
10. Ochoa MC, et al. Enhancement of antibody-dependent cellular cytotoxicity of cetuximab by a chimeric protein encompassing interleukin-15. Oncoimmunology 1017; 7(2):eprint.
11. Godfrey, D.I., et al, Raising the NKT cell family. Nat Immunol. 2010 Mar; 11(3):197-206.

Although the present invention and its advantages have been described in detail, it should be understood that various changes, substitutions and alterations can be made herein without departing from the spirit and scope of the invention as defined by the appended claims. Moreover, the scope of the present application is not intended to be limited to the particular embodiments of the process, machine, manufacture, composition of matter, means, methods and steps described in the specification. As one of ordinary skill in the art will readily appreciate from the disclosure of the present invention, processes, machines, manufacture, compositions of matter, means, methods, or steps, presently existing or later to be developed that perform substantially the same function or achieve substantially the same result as the corresponding embodiments described herein may be utilized according to the present invention. Accordingly, the appended claims are intended to include within their scope such processes, machines, manufacture, compositions of matter, means, methods, or steps.

### Embodiments of the invention:

1. An immune effector cell, wherein the cell produces a molecular adaptor comprising:
   (a) a tumor cell binding domain or target cell binding domain, and
   (b) a cytokine domain comprising
      (i) a cytokine or a functional fragment thereof; or
      (ii) a cytokine receptor or a functional fragment thereof; or
      (iii) a combination of both (i) and (ii);
   (c) optionally further comprising an antigen receptor target domain;
   optionally wherein the cell comprises one or more engineered antigen receptors against one or more tumor target antigens or target antigens.
2. The cell of item 1, wherein the tumor cell binding domain comprises an antibody, an antibody fragment or a non-antibody scaffold.
3. The cell of item 1 or 2, wherein the tumor cell binding domain comprises an scFv.
4. The cell of item 1, 2, or 3, wherein the tumor cell binding domain targets an antigen expressed on the surface of a tumor cell, such as GD2, BCMA, CD56, CD24, L1CAM, GPC2, GPC3, CSPG4, HER2, CD19, CD22, CD30, CD71, B7-H3, B7-H4, PD-L1, or PDL-2.
5. The cell of any one of the preceding items, wherein said cytokine is selected from the group consisting of IL-7, IL-12, IL-15, IL-18, IL-27, IL-33, or IL-21.
6. The cell of any one of items 1-5, wherein the cytokine is IL-15 and/or the cytokine receptor is IL-15R or the alpha subunit thereof (IL15RA).
7. The cell of any one of the preceding items, wherein the antigen receptor target domain comprises a tumor antigen or a functional fragment thereof.
8. The cell of item 7, wherein the molecular adaptor comprises
   a tumor cell binding domain targeting a first antigen on the tumor cell and
   an antigen receptor target domain comprising a second tumor antigen or functional fragment thereof.
9. The cell of any one of the preceding items, wherein the antigen receptor target domain comprises CD 19.
10. The cell of any one of the preceding items, wherein the one or more engineered antigen receptors is a non-natural T cell receptor (TCR) or a chimeric antigen receptor (CAR).
11. The cell of anyone of the preceding items, wherein said cell
   produces a molecular adaptor targeting a first tumor antigen and comprises an antigen receptor targeting a first tumor antigen;
   produces a molecular adaptor targeting a second tumor antigen and comprises an antigen receptor targeting a first tumor antigen; or
   produces a molecular adaptor targeting a first tumor antigen, said molecular adaptor comprising an antigen receptor target domain that is targeted by an antigen receptor expressed on the surface of the cell.
12. The cell of any one of the preceding items, wherein the tumor cell binding domain and the antigen receptor target domain are linked by a hinge region and/or linker.
13. The cell of item 12, wherein the hinge region is from IgG1, IgG2, IgG3, or IgG4.
14. The cell of any one of the preceding items, comprising an expression vector or RNA encoding said molecular adaptor and optionally said engineered antigen receptor.
15. The cell of item 14, wherein the expression vector is a viral vector or a non-viral vector.
16. The cell of item 15, wherein the viral vector is a retroviral, lentiviral, adenoviral, or adeno-associated viral vector.
17. The cell of any one of items 1-16, wherein the cell is an NK cell, an NKT cell, a T cell, a γδ T cell, a Mucosal-associated invariant T (MAIT) cell, a macrophage, an innate lymphoid (IL) cell, cytokine-induced killer (CIK) cell, stem cell or a mixture thereof.
18. A molecular adaptor for redirecting cells expressing engineered antigen receptors, comprising:
   (a) a tumor cell binding domain,
   (b) a cytokine domain comprising
      (i) a cytokine or a functional fragment thereof; or
      (ii) a cytokine receptor or a functional fragment thereof; or
      (iii) a combination of both (i) and (ii).
19. The molecular adaptor of item 18, wherein the molecular adaptor further comprises (c) an antigen receptor target domain.
20. The molecular adaptor of item 18 or 19, wherein the cytokine domain in the molecular adaptor is positioned between the tumor cell binding domain and the antigen receptor domain.
21. A non-natural antigen receptor directed against the molecular adaptor of any one of items 18-20.
22. A polynucleotide encoding the molecular adaptor of any one of items 18-20.
23. An expression vector comprising the polynucleotide of item 22.
24. The expression vector of item 23, further comprising a sequence that encodes an antigen receptor.
25. An expression vector encoding:
   a molecular adaptor comprising:
   (i) a tumor cell binding domain, and
   (ii) a cytokine domain; and
   (b) an antigen receptor.
26. The expression vector of any one of items 24-25, wherein an IRES element or a 2A sequence is located between the molecular adaptor and the antigen receptor.
27. The expression vector of any one of items 23-26, wherein said expression vector is a viral vector or a non-viral vector.
28. The expression vector of item 27, wherein the viral vector is a retroviral, lentiviral, adenoviral, or adeno-associated viral vector.
29. A cell comprising the expression vector of any one of items 21-26.
30. A method of treating cancer in an individual, comprising the step of administering to the individual a therapeutically effective amount of the cells of any one of items 1-17 or 29.
31. A method of treating cancer in an individual, comprising administering a therapeutically effective amount of the molecular adaptor of any one of items 18-20 and/or a therapeutically effective amount of cells expressing an antigen receptor of item 21.
32. A method of treating cancer in an individual, comprising administering to the individual a therapeutically effective amount of:
   a molecular adaptor comprising a tumor cell binding domain targeting a first or a second antigen on a tumor cell and a cytokine domain, and
   cells expressing an antigen receptor against a first antigen on the tumor cell.
33. The method of any one of items 31-32, wherein the molecular adaptor and/or the cells are administered to the individual by intraperitoneal or intravenous injection.
34. The method of items 31-33, wherein a second dose of the molecular adaptor is administered after an initial dose.
35. The method of any one of items 30-34, wherein the individual is receiving, has received, and/or will receive an additional cancer therapy.
36. The method of item 35, wherein the additional cancer therapy is surgery, radiation, chemotherapy, immunotherapy, hormone therapy, or a combination thereof.
37. A pharmaceutical composition comprising the immune effector cell of items 1-17 or the molecular adaptor of any one of items 18-20.
38. A method of adapting a cancer therapy to a cancer expressing a first tumor antigen, comprising the steps of:
   (a) providing or obtaining an immune effector cell or plurality thereof comprising a non-natural antigen receptor that targets a second tumor antigen; and
   (b) providing or obtaining a molecular adaptor comprising a tumor cell binding domain that binds the first tumor antigen and an antigen receptor target domain that binds the antigen receptor that targets a second tumor antigen,
   optionally wherein the molecular adaptor comprises (i) a cytokine or a functional fragment thereof; or (ii) a cytokine receptor or a functional fragment thereof; or (iii) a combination of both (i) and (ii),
   and wherein the providing or obtaining of the molecular adaptor in step (b) is determined by the presence of expression of the first antigen in the cancer cells of the cancer being treated.
39. A method of adapting a cancer therapy to a cancer expressing a first tumor antigen, comprising the steps of:
   (a) providing or obtaining an immune effector cell or plurality thereof comprising a non-natural antigen receptor that targets a second tumor antigen; and
   (b) transfecting said cells with a polynucleotide that encodes a molecular adaptor comprising:
      (1) a tumor cell binding domain that targets the first antigen, and
      (2) a cytokine domain comprising:
         (i) a cytokine or a functional fragment thereof; or
         (ii) a cytokine receptor or a functional fragment thereof; or
         (iii) a combination of both (i) and (ii).

## Claims

1. An immune effector cell, wherein the cell produces a molecular adaptor comprising:
(a) a tumor cell binding domain or target cell binding domain, and
(b) a cytokine domain comprising
(i) a cytokine or a functional fragment thereof; or
(ii) a cytokine receptor or a functional fragment thereof; or
(iii) a combination of both (i) and (ii);
(c) optionally further comprising an antigen receptor target domain;
optionally wherein the cell comprises one or more engineered antigen receptors against one or more tumor target antigens or target antigens.

2. The cell of claim 1, wherein:
(A) the tumor cell binding domain comprises an antibody, an antibody fragment or a non-antibody scaffold;
(B) the tumor cell binding domain comprises an scFv;
(C) the tumor cell binding domain targets an antigen expressed on the surface of a tumor cell, such as GD2, BCMA, CD56, CD24, L1CAM, GPC2, GPC3, CSPG4, HER2, CD19, CD22, CD30, CD71, B7-H3, B7-H4, PD-L1, or PDL-2;
(D) said cytokine is selected from the group consisting of IL-7, IL-12, IL-15, IL-18, IL-27, IL-33, or IL-21;
(E) the cytokine is IL-15 and/or the cytokine receptor is IL-15R or the alpha subunit thereof (IL15RA);
(F) the antigen receptor target domain comprises a tumor antigen or a functional fragment thereof, optionally wherein the molecular adaptor comprises
a tumor cell binding domain targeting a first antigen on the tumor cell and
an antigen receptor target domain comprising a second tumor antigen or functional fragment thereof;
(G) the antigen receptor target domain comprises CD19;
(H) the one or more engineered antigen receptors is a non-natural T cell receptor (TCR) or a chimeric antigen receptor (CAR);
(I) said cell
produces a molecular adaptor targeting a first tumor antigen and comprises an antigen receptor targeting a first tumor antigen;
produces a molecular adaptor targeting a second tumor antigen and comprises an antigen receptor targeting a first tumor antigen; or
produces a molecular adaptor targeting a first tumor antigen, said molecular adaptor comprising an antigen receptor target domain that is targeted by an antigen receptor expressed on the surface of the cell;
(J) the tumor cell binding domain and the antigen receptor target domain are linked by a hinge region and/or linker, optionally wherein the hinge region is from IgG1, IgG2, IgG3, or IgG4; and/or
(K) the cell is an NK cell, an NKT cell, a T cell, a γδ T cell, a Mucosal-associated invariant T (MAIT) cell, a macrophage, an innate lymphoid (IL) cell, cytokine-induced killer (CIK) cell, stem cell or a mixture thereof.

3. The cell of any one of the preceding claims, comprising an expression vector or RNA encoding said molecular adaptor and optionally said engineered antigen receptor, optionally wherein the expression vector is a viral vector or a non-viral vector and optionally wherein the viral vector is a retroviral, lentiviral, adenoviral, or adeno-associated viral vector.

4. A molecular adaptor for redirecting cells expressing engineered antigen receptors, comprising:
(a) a tumor cell binding domain,
(b) a cytokine domain comprising
(i) a cytokine or a functional fragment thereof; or
(ii) a cytokine receptor or a functional fragment thereof; or
(iii) a combination of both (i) and (ii).

5. The molecular adaptor of claim 18, wherein:
(A) the molecular adaptor further comprises (c) an antigen receptor target domain; and/or
(B) the cytokine domain in the molecular adaptor is positioned between the tumor cell binding domain and the antigen receptor domain.

6. A non-natural antigen receptor directed against the molecular adaptor of claim 4 or 5.

7. A polynucleotide encoding the molecular adaptor of claim 4 or 5.

8. An expression vector comprising the polynucleotide of claim 7, optionally wherein the expression vector further comprises a sequence that encodes an antigen receptor and optionally wherein an IRES element or a 2A sequence is located between the molecular adaptor and the antigen receptor.

9. An expression vector encoding:
a molecular adaptor comprising:
(i) a tumor cell binding domain, and
(ii) a cytokine domain; and
(b) an antigen receptor,
optionally wherein an IRES element or a 2A sequence is located between the molecular adaptor and the antigen receptor.

10. The expression vector of claim 8 or 9, wherein said expression vector is a viral vector or a non-viral vector, optionally wherein the viral vector is a retroviral, lentiviral, adenoviral, or adeno-associated viral vector.

11. A cell comprising the expression vector of any one of claims 8-10.

12. A method of treating cancer in an individual, comprising:
(a) the step of administering to the individual a therapeutically effective amount of the cells of any one of claims 1-3 or 11; or
(b) administering a therapeutically effective amount of the molecular adaptor of claim 4 or 5 and/or a therapeutically effective amount of cells expressing an antigen receptor of claim 6; or
(c) administering to the individual a therapeutically effective amount of:
(i) a molecular adaptor comprising a tumor cell binding domain targeting a first or a second antigen on a tumor cell and a cytokine domain, and
(ii) cells expressing an antigen receptor against a first antigen on the tumor cell;
optionally wherein the individual is receiving, has received, and/or will receive an additional cancer therapy and optionally wherein the additional cancer therapy is surgery, radiation, chemotherapy, immunotherapy, hormone therapy, or a combination thereof.

13. The method of claim 12(b) or 12(c), wherein:
(A) the molecular adaptor and/or the cells are administered to the individual by intraperitoneal or intravenous injection; and/or
(B) a second dose of the molecular adaptor is administered after an initial dose.

14. A pharmaceutical composition comprising the immune effector cell of claims 1-3 or the molecular adaptor of claim 4 or 5.

15. A method of adapting a cancer therapy to a cancer expressing a first tumor antigen, comprising the steps of:
(A)
(a) providing or obtaining an immune effector cell or plurality thereof comprising a non-natural antigen receptor that targets a second tumor antigen; and
(b) providing or obtaining a molecular adaptor comprising a tumor cell binding domain that binds the first tumor antigen and an antigen receptor target domain that binds the antigen receptor that targets a second tumor antigen,
optionally wherein the molecular adaptor comprises (i) a cytokine or a functional fragment thereof; or (ii) a cytokine receptor or a functional fragment thereof; or (iii) a combination of both (i) and (ii),
and wherein the providing or obtaining of the molecular adaptor in step (b) is determined by the presence of expression of the first antigen in the cancer cells of the cancer being treated; or
(B)
(a) providing or obtaining an immune effector cell or plurality thereof comprising a non-natural antigen receptor that targets a second tumor antigen; and
(b) transfecting said cells with a polynucleotide that encodes a molecular adaptor comprising:
(1) a tumor cell binding domain that targets the first antigen, and
(2) a cytokine domain comprising:
(i) a cytokine or a functional fragment thereof; or
(ii) a cytokine receptor or a functional fragment thereof; or
(iii) a combination of both (i) and (ii).
